# EUROPÄISCHE PATENTSCHRIFT

(11) **EP 3 631 014 B1**
(45) Veröffentlichungstag und Bekanntmachung des Hinweises auf die Patenterteilung: **11.05.2022**
(21) Anmeldenummer: 18726152.4
(22) Anmeldetag: 22.05.2018
(51) Int. Cl.: C12Q 1/6876, C12Q 1/6827, C12Q 1/6888

(54) **METHODE ZUR GENOTYPISIERUNG VON MAUSSTÄMMEN**
METHOD FOR THE GENOTYPING OF MOUSE STRAINS
MÉTHODE DE GÉNOTYPAGE DE SOUCHES SUR SOURIS

(30) Priorität: 24.05.2017 DE 102017005000
(43) Veröffentlichungstag der Anmeldung: 08.04.2020
(73) Patentinhaber: GVG Genetic Monitoring GmbH, 04103 Leipzig (DE)
(72) Erfinder: DOBROWOLSKI, Peter, 01445 Radebeul (DE); FISCHER, Melina, 17121 Rustow, Stadt Loitz (DE)
(74) Vertreter: Maikowski & Ninnemann Patentanwälte Partnerschaft mbB
(86) Internationale Anmeldenummer: PCT/EP2018/063343
(87) Internationale Veröffentlichungsnummer: WO 2018/215436

(56) Entgegenhaltungen:
- WO-A1-2016/008894
- US-A1- 2014 066 322
- Dirk Wedekind ET AL: "Autoren: Reinhart Kluge, Potsdam-Rehbrücke", , 1. Januar 2016 (2016-01-01), XP055490163, Gefunden im Internet: URL:http://www.gv-solas.de/fileadmin/user_ upload/pdf_publikation/Genetik/201605Auszu chtstaemme_.pdf [gefunden am 2018-07-04] in der Anmeldung erwähnt
- BINNAZ YALCIN ET AL: "Commercially Available Outbred Mice for Genome-Wide Association Studies", PLOS GENETICS, Bd. 6, Nr. 9, 2. September 2010 (2010-09-02), Seite e1001085, XP055489824, US ISSN: 1553-7390, DOI: 10.1371/journal.pgen.1001085 in der Anmeldung erwähnt
- OLGA A IAKOUBOVA ET AL: "Microsatellite marker panels for use in high-throughput genotyping of mouse crosses", PHYSIOLOGICAL GENOMICS, Bd. 3, Nr. 3, 8. September 2000 (2000-09-08), Seiten 145-148, XP055145039, ISSN: 1094-8341
- BINNAZ YALCIN ET AL: "Association studies in outbred mice in a new era of full-genome sequencing", MAMMALIAN GENOME, SPRINGER-VERLAG, NE, Bd. 23, Nr. 9 - 10, 31. Juli 2012 (2012-07-31), Seiten 719-726, XP035120573, ISSN: 1432-1777, DOI: 10.1007/S00335-012-9409-Z

### SEQUENZPROTOKOLL

<110> GVG Genetic Monitoring GmbH, 04103 Leipzig, DE
<120> Methode zur Genotypisierung von Mausstämmen
<130> GVG101WO
<150> DE 10 2017 005 000.7
   <151> 2017-05-24
<160> 76
<170> PatentIn version 3.5
<210> 1
   <211> 22
   <212> DNA
   <213> Artifizielle Sequenz
<220>
   <223> Synthetisches Oligonukleotid
<400> 1
   caaaaggagg cgagtagggt ga 22
<210> 2
   <211> 23
   <212> DNA
   <213> Artifizielle Sequenz
<220>
   <223> Synthetisches Oligonukleotid
<400> 2
   atagtacgtg gcacaatggg aga 23
<210> 3
   <211> 23
   <212> DNA
   <213> Artifizielle Sequenz
<220>
   <223> synthetic olignucleotide
<400> 3
   taacaagcag caatgatgag tgc 23
<210> 4
   <211> 20
   <212> DNA
   <213> Artifizielle Sequenz
<220>
   <223> Synthetisches Oligonukleotid
<400> 4
   aagtgatctt acagccaaca 20
<210> 5
   <211> 22
   <212> DNA
   <213> Artifizielle Sequenz
<220>
   <223> Synthetisches Oligonukleotid
<400> 5
   gtggctctta tccagaacct ag 22
<210> 6
   <211> 23
   <212> DNA
   <213> Artifizielle Sequenz
<220>
   <223> Synthetisches Oligonukleotid
<400> 6
   ttctttctcc acacatccac tgc 23
<210> 7
   <211> 22
   <212> DNA
   <213> Artifizielle Sequenz
<220>
   <223> Synthetisches Oligonukleotid
<400> 7
   caaactgagt gtgacgtagg ac 22
<210> 8
   <211> 21
   <212> DNA
   <213> Artifizielle Sequenz
<220>
   <223> Synthetisches Oligonukleotid
<400> 8
   agtcccagca gcaacagaga a 21
<210> 9
   <211> 21
   <212> DNA
   <213> Artifizielle Sequenz
<220>
   <223> Synthetisches Oligonukleotid
<400> 9
   tgtgatacaa aggctgccag g 21
<210> 10
   <211> 21
   <212> DNA
   <213> Artifizielle Sequenz
<220>
   <223> Synthetisches Oligonukleotid
<400> 10
   accagcaggt gtttgggaga a 21
<210> 11
   <211> 25
   <212> DNA
   <213> Artifizielle Sequenz
<220>
   <223> Synthetisches Oligonukleotid
<400> 11
   tctggagctc aaagaaacat agagt 25
<210> 12
   <211> 23
   <212> DNA
   <213> Artifizielle Sequenz
<220>
   <223> Synthetisches Oligonukleotid
<400> 12
   cagggctaaa tagtaagaca gag 23
<210> 13
   <211> 23
   <212> DNA
   <213> Artifizielle Sequenz
<220>
   <223> Synthetisches Oligonukleotid
<400> 13
   cttggacctt catattcagt gtg 23
<210> **14**
   <211> 23
   <212> DNA
   <213> Artifizielle Sequenz
<220>
   <223> Synthetisches Oligonukleotid
<400> **14**
   tccagagttt caaatcctcc ctc 23
<210> 15
   <211> 19
   <212> DNA
   <213> Artifizielle Sequenz
<220>
   <223> Synthetisches Oligonukleotid
<400> 15
   ctagacaggg gatctggct 19
<210> 16
   <211> 21
   <212> DNA
   <213> Artifizielle Sequenz
<220>
   <223> Synthetisches Oligonukleotid
<400> 16
   cccccttctg aaactgtgtc c 21
<210> 17
   <211> 23
   <212> DNA
   <213> Artifizielle Sequenz
<220>
   <223> Synthetisches Oligonukleotid
<400> 17
   tagatctgtg cactaagtcc gac 23
<210> 18
   <211> 21
   <212> DNA
   <213> Artifizielle Sequenz
<220>
   <223> Synthetisches Oligonukleotid
<400> 18
   tctgacatct gagagcagtc a 21
<210> 19
   <211> 24
   <212> DNA
   <213> Artifizielle Sequenz
<220>
   <223> Synthetisches Oligonukleotid
<400> 19
   gctagaaatg agtacgtaga tcac 24
<210> 20
   <211> 21
   <212> DNA
   <213> Artifizielle Sequenz
<220>
   <223> Synthetisches Oligonukleotid
<400> 20
   agggccagcc tctgctatgt c 21
<210> 21
   <211> 22
   <212> DNA
   <213> Artifizielle Sequenz
<220>
   <223> Synthetisches Oligonukleotid
<400> 21
   cagttgctga taacctgaca gc 22
<210> 22
   <211> 22
   <212> DNA
   <213> Artifizielle Sequenz
<220>
   <223> Synthetisches Oligonukleotid
<400> 22
   cccagtgttc ctgaccaatg tt 22
<210> 23
   <211> 21
   <212> DNA
   <213> Artifizielle Sequenz
<220>
   <223> Synthetisches Oligonukleotid
<400> 23
   caagcaaggt ccaggtcact a 21
<210> 24
   <211> 22
   <212> DNA
   <213> Artifizielle Sequenz
<220>
   <223> Synthetisches Oligonukleotid
<400> 24
   caatgccaca ctcacctaac ac 22
<210> 25
   <211> 23
   <212> DNA
   <213> Artifizielle Sequenz
<220>
   <223> Synthetisches Oligonukleotid
<400> 25
   aataagttct tgttcctttc ctc 23
<210> 26
   <211> 25
   <212> DNA
   <213> Artifizielle Sequenz
<220>
   <223> Synthetisches Oligonukleotid
<400> 26
   tttggctgcc ctaaactctt tgtag 25
<210> 27
   <211> 21
   <212> DNA
   <213> Artifizielle Sequenz
<220>
   <223> Synthetisches Oligonukleotid
<400> 27
   tcactccacc ttgagaactc t 21
<210> 28
   <211> 22
   <212> DNA
   <213> Artifizielle Sequenz
<220>
   <223> Synthetisches Oligonukleotid
<400> 28
   gctcagtgaa ctcagtccta ag 22
<210> 29
   <211> 22
   <212> DNA
   <213> Artifizielle Sequenz
<220>
   <223> Synthetisches Oligonukleotid
<400> 29
   caaggacact gagagcaaca ga 22
<210> 30
   <211> 23
   <212> DNA
   <213> Artifizielle Sequenz
<220>
   <223> Synthetisches Oligonukleotid
<400> 30
   gaagaggact atgactggct gtt 23
<210> 31
   <211> 22
   <212> DNA
   <213> Artifizielle Sequenz
<220>
   <223> Synthetisches Oligonukleotid
<400> 31
   cgcgactgaa atgtcctgaa tg 22
<210> 32
   <211> 25
   <212> DNA
   <213> Artifizielle Sequenz
<220>
   <223> Synthetisches Oligonukleotid
<400> 32
   acatgaaggc ttacaaccat ctgaa 25
<210> 33
   <211> 21
   <212> DNA
   <213> Artifizielle Sequenz
<220>
   <223> Synthetisches Oligonukleotid
<400> 33
   tacagcggca aagagaggaa g 21
<210> 34
   <211> 21
   <212> DNA
   <213> Artifizielle Sequenz
<220>
   <223> Synthetisches Oligonukleotid
<400> 34
   aagagagacc ccttggtact g 21
<210> 35
   <211> 22
   <212> DNA
   <213> Artifizielle Sequenz
<220>
   <223> Synthetisches Oligonukleotid
<400> 35
   gaatggatct gcatctgagg tt 22
<210> 36
   <211> 23
   <212> DNA
   <213> Artifizielle Sequenz
<220>
   <223> Synthetisches Oligonukleotid
<400> 36
   ctcgaactta gacaactgtg gtc 23
<210> 37
   <211> 21
   <212> DNA
   <213> Artifizielle Sequenz
<220>
   <223> Synthetisches Oligonukleotid
<400> 37
   aggcctacag acagttgtgc a 21
<210> 38
   <211> 21
   <212> DNA
   <213> Artifizielle Sequenz
<220>
   <223> Synthetisches Oligonukleotid
<400> 38
   cttccatgct agggctagtg a 21
<210> 39
   <211> 21
   <212> DNA
   <213> Artifizielle Sequenz
<220>
   <223> Synthetisches Oligonukleotid
<400> 39
   cccttaatca attgctgcaa a 21
<210> 40
   <211> 20
   <212> DNA
   <213> Artifizielle Sequenz
<220>
   <223> Synthetisches Oligonukleotid
<400> 40
   agccacatgt gcttgctttc 20
<210> 41
   <211> 18
   <212> DNA
   <213> Artifizielle Sequenz
<220>
   <223> Synthetisches Oligonukleotid
<400> 41
   ctgagtgatg cagggtgc 18
<210> 42
   <211> 20
   <212> DNA
   <213> Artifizielle Sequenz
<220>
   <223> Synthetisches Oligonukleotid
<400> 42
   ggctgtgaat ccagaggctt 20
<210> 43
   <211> 20
   <212> DNA
   <213> Artifizielle Sequenz
<220>
   <223> Synthetisches Oligonukleotid
<400> 43
   tgtagtcccc tggatcccat 20
<210> **44**
   <211> 22
   <212> DNA
   <213> Artifizielle Sequenz
<220>
   <223> Synthetisches Oligonukleotid
<400> **44**
   tgccagctca agttgtcttt tg 22
<210> 45
   <211> 20
   <212> DNA
   <213> Artifizielle Sequenz
<220>
   <223> Synthetisches Oligonukleotid
<400> 45
   gtcagaaggg gcagactcta 20
<210> 46
   <211> 20
   <212> DNA
   <213> Artifizielle Sequenz
<220>
   <223> Synthetisches Oligonukleotid
<400> 46
   tcacttggga tggtgaactg 20
<210> 47
   <211> 21
   <212> DNA
   <213> Artifizielle Sequenz
<220>
   <223> Synthetisches Oligonukleotid
<400> 47
   cagacctcag ggcatttcca t 21
<210> 48
   <211> 21
   <212> DNA
   <213> Artifizielle Sequenz
<220>
   <223> Synthetisches Oligonukleotid
<400> 48
   atatggctct ttgttctctg g 21
<210> 49
   <211> 20
   <212> DNA
   <213> Artifizielle Sequenz
<220>
   <223> Synthetisches Oligonukleotid
<400> 49
   cagatctcag ggcattccca 20
<210> 50
   <211> 21
   <212> DNA
   <213> Artifizielle Sequenz
<220>
   <223> Synthetisches Oligonukleotid
<400> 50
   atatggctct ttgttctctg g 21
<210> 51
   <211> 176
   <212> DNA
   <213> Mus musculus
<400> 51
   caaaaggagg cgagtagggt gacattactg tcctaaaaat caagactctt tctttctttc        60
   tttctttctt tctttctttc tttctttctt tctttctttc tttctttctt tctttctttc       120
   tttctttctt tctttctatc acagaaaaaa tgctctccca ttgtgccacg tactat           176
<210> 52
   <211> 503
   <212> DNA
   <213> Mus musculus
<400> 52
   taacaagcag caatgatgag tgctttgcaa atatatctgg gctattctaa aattgactac        60
   tataaaccac cccatctatc tagacagttt tgtttcctgg agggcattct gttttgttct       120
   gtaatgatat ttcaagctgt atgggagtct tgtttccaca cgtgactaca tttattaact       180
   tagccacatc taaaatacag tgtgggaacc cagtaagggt atatggtatc ttaactctca       240
   tccaattttc agctgggagc attgcatgct attttcccct tgttttcctt tctttctttc       300
   tttctttctt tctttctttc tttctttctt tctttctttc tttctttctt tctttctttc       360
   taatgtttct tttcaaagat tttgaatttg taatgaacac atgagggtat atatcccacc       420
   ttgatctagc tgtggagatc cccttggcta ctgtggaaaa taccactaga caactgtggg       480
   agatgttggc tgtaagatca ctt                                               503
<210> 53
   <211> 484
   <212> DNA
   <213> Mus musculus
<400> 53
   gtggctctta tccagaacct agggaggatt taagacattt ggaaggtcca aatgcttagc        60
   tgcaagcatt gcacccattt ctccctttct ttctttcttt ctttctttct ttctttcttt       120
   ctttctttct ttctttcttt ctttctttct ttctttcttt ctttctttct ttctttctaa       180
   ttttttatta ggtattttcc tcatttacat ttctaatgct atcccaaaag tcccccatac       240
   ccaccccccc ccactcccct acccaccaac tctccctttt tggccctggc gttcccctgt       300
   actggggcat ataaagtttt gcacccattt cttataagga acttcagcat ttgtggattt       360
   tggtttgtgg tatacaggat cttggaagaa tcccttagac accaaggaac tactgactac       420
   tggacagagt attttgtatg tgtagtagct aggtacccaa ggcagtggat gtgtggagaa       480
   agaa                                                                    484
<210> 54
   <211> 180
   <212> DNA
   <213> Mus musculus
<400> 54
   caaactgagt gtgacgtagg acaggaataa agatcagacc gcgaggcaag gaggcaaaag 60
   aaagaaagaa agaaagaaag aaagaaagaa agaaagaaag aaagaaagaa agaaagaaag 120
   aaagaaagaa agaaagaaaa gggagagaat ctggtggttt tctctgttgc tgctgggact 180
<210> 55
   <211> 317
   <212> DNA
   <213> Mus musculus
<400> 55
   tgtgatacaa aggctgccag gcttcagtag agtatatttg ccttcctggg tgcctattct 60
   gttttccttt ctttctttct ttctttcttt ctttctttct ttctttcttt ctttctttct 120
   ttctttcttt ctttctttct ttctctgaga cagggtttct ctgtgtagcc ctggctgtcc 180
   tggaactcgc tttgtagacc aggctggcct cgaactcagt aatccacctg cctctgcctc 240
   ccaaatgctg ggattaaagg cgataacccc tttcaacctc attctttgca tgccccttct 300
   cccaaacacc tgctggt 317
<210> 56
   <211> 135
   <212> DNA
   <213> Mus musculus
<400> 56
   tctggagctc aaagaaacat agagttattg caattaattt ctttctttct ttctttcttt 60
   ctttctttct ttctttcttt ctttctttct ttctttcgag agacaggtct tactctgtct 120
   tactatttag ccctg 135
<210> 57
   <211> **144**
   <212> DNA
   <213> Mus musculus
<400> 57
   cttggacctt catattcagt gtgggaaaga aagaaagaaa gaaagaaaga aagaaagaaa 60
   gaaagaaaga aagaaagaaa gaaagaaaga aagaaagaaa gaaagaaaga aaggtaaaaa 120
   agagggagga tttgaaactc tgga 144
<210> 58
   <211> 216
   <212> DNA
   <213> Mus musculus
<400> 58
   ctagacaggg gatctggcta ggaggagtgt ttgcagaaga aacaggcatt aactcttaac 60
   tcttgcccag gccactctgt ttctagctgg gatgacacca tcagaattct ttctttcttt 120
   ctttctttct ttctttcttt ctttctttct ttctttcttt ctttctttct ttctttcttt 180
   ctttcttctt cttttggaca cagtttcaga aggggg 216
<210> 59
   <211> 399
   <212> DNA
   <213> Mus musculus
<400> 59
   tagatctgtg cactaagtcc gacacacaaa taaaatacag aacgaaaagc ccagaggaga 60
   gaaagaatcc tcaagaaaca tccaagaaga cattgcagag tgtgaagagt gaagggctct 120
   agcccaggcc cacatcatgg tgtatctagg gaaagtttta tgagcttcta gaaagaaaac 180
   aaacacaatc aagacataca tttatttgtt tattctttct ttctttctct ctccttcttt 240
   ctttctttct ttctttcttt ctttctttct ttctttcttt ctttctttct ttctttcttt 300
   ctttctttct tcattaattc attcatttat gtgtgtggct gtcttgcctg catggttatc 360
   tttataccac atgtgcactg actgctctca gatgtcaga 399
<210> 60
   <211> 330
   <212> DNA
   <213> Mus musculus
<400> 60
   agggccagcc tctgctatgt cgggaatttt atgagacctt gtcagaaaga aagaaagaaa 60
   gaaagaaaga aagaaagaaa gaaagaaaga aagaaagaaa gaaagaaaga aagaaagaaa 120
   gaaagggaaa gaaagaaacc ataggaagta atttgtgcat taaaatgtgt gttaagtgaa 180
   tgtagtcatt tatccaaccc atatttaggt ctcatttata aaaacagtga cccagaatat 240
   gcatctaacg tgagtagcct gaagccgaag cgcctgtgta cagagtgggt tgagagagga 300
   tacttggtga tctacgtact catttctagc 330
<210> 61
   <211> 393
   <212> DNA
   <213> Mus musculus
<400> 61
   cagttgctga taacctgaca gccaaaattc cacagagcca cataacagaa gaagtgaaca 60
   tacatttaca tgcatagtca cttaacacac ttaaatagaa aagaaagaaa gaaagaaaga 120
   aagaaagaaa gaaagaaaga aagaaagaaa gaaagaaaga aagaaagaaa gaaagaaagg 180
   aaatagagaa aagagattgc caatatgaaa gaagtgactc tagcccattc aaaactgtat 240
   aaaagaatgt ctggagcctg gctgggggaa tggataccaa tcatgccaac atcctagaaa 300
   cctttgctgg agccactgac acacccacca agaaacaagc ttcttactgg gtgcttctgg 360
   cctgaggcat aaacattggt caggaacact ggg 393
<210> 62
   <211> 292
   <212> DNA
   <213> Mus musculus
<400> 62
   caatgccaca ctcacctaac acaggcgtgc tgtagaggaa ctcaactcga ggtccagcct 60
   ttgcctgaaa acttccccca gacagctgca agaactcaca gtactggttt ttgtgtgtct 120
   ttctttcttt tctttctttc tttctttctt tctttctttc tttctttctt tctttctttc 180
   tttctttctt tctttccttc tttctttttc ttatccatgt ccaggagctg tcctggacac 240
   tggctgcttc ccaaggagct tcggtagggc atagtgacct ggaccttgct tg 292
<210> 63
   <211> 136
   <212> DNA
   <213> Mus musculus
<400> 63
   tttggctgcc ctaaactctt tgtagtgccc aattgaagaa agaaagaaag aaagaaagaa 60
   agaaagaaag aaagaaagaa agaaagaaag aaagaaagaa agaaagaaag aaagaggaaa 120
   ggaacaagaa cttatt 136
<210> 64
   <211> 308
   <212> DNA
   <213> Mus musculus
<400> 64
   tcactccacc ttgagaactc tctttgtgcc tattagattt ttcattttag atctttcttc 60
   atttttatac cagtgttgtg gttgagtttt gtttctattg ttttttccat gaatttgacc 120
   aagaaaagcc ctgggaaaga atttctttct ttctttcttt ctttctttct ttctttcttt 180
   ctttctttct ttctttcttt ctttctttct tttttttttt taagctagct gcatacactt 240
   gcatatcttg ccctaggctg tgggacaata aacttacttt tctaacctta ggactgagtt 300
   cactgagc 308
<210> 65
   <211> 505
   <212> DNA
   <213> Mus musculus
<400> 65
   caaggacact gagagcaaca gagaggccct ctgttgctga ggagtctcta tcccaactca 60
   gcccccaaca ctgagcatct ccctcacaat ttccatccca gacccccata ataacaggag 120
   gggcctaggg agcccttcct actctcttga ataccaccaa taaagttcgc tgcacagaaa 180
   gaaagaagga aagaaagaaa gaaagaaaga aagaaagaaa gaaagaaaga aagaaagaaa 240
   gaaagaaaga aagaaagagc taacctctgg tatttcccca gactgaactc tcagagttgg 300
   aagagactcc caaggtcaca ttgcccattc tcttacttta tggataagaa agggtggttc 360
   agagaacttg aaatagccct caggcagtgc gtggcaggag gattttgacc tcaacctgat 420
   cttttctcca taatcagtgg gcagtgatct accaccacct tctgcttgtg aggcagacat 480
   gaaacagcca gtcatagtcc tcttc 505
<210> 66
   <211> 245
   <212> DNA
   <213> Mus musculus
<400> 66
   acatgaaggc ttacaaccat ctgaacagct acagtatact cacatacaat aaataaataa 60
   ataaataaat aaataaacaa ataaataaat cttttaagaa agaaagagag agaggaagaa 120
   aggaagaaag aaagaaagaa agaaagaaag aaagaaagaa agaaagaaag aaagaaagaa 180
   agaaagaaac aggatttgta tctagaaatg cctacatgag ttgcattcag gacatttcag 240
   tcgcg 245
<210> 67
   <211> 396
   <212> DNA
   <213> Mus musculus
<400> 67
   tacagcggca aagagaggaa gaaagaaaga aagaaagaaa gaaagaaaga aagaaagaaa        60
   gaaagaaaga aagaaagaaa gaaagacaga caaatgcaga tttctaaaat ttaggagtaa       120
   ctgaagtata tttttaaaat gcaaaattgg tcctgttttt atgggaacac taatggcaac       180
   tcaaccccag ataatttttc ttatttattt tatctatcta tctatctatt tatttattta       240
   tttatttatt actagatatt ttctttatat acatttcaaa tgctatcctg aaagttccct       300
   atactctccc tctgccctgc tcccctaacc acccactccc gcttcttggc cctgtactgg       360
   ggcatataaa gtttgcagta ccaaggggtc tctctt                                 396
<210> 68
   <211> 283
   <212> DNA
   <213> Mus musculus
<400> 68
   gaatggatct gcatctgagg ttttcttcct tcctccctcc ctccctccct tgcttctttc        60
   tttctttctt tctttctttc tttctttctt tctttctttc tttctttctt tctttctttc       120
   tttctttctt tctttctttc cttcatgtat cctgtctgtt tttaagatta gccacataga       180
   gagcaatttg ataacagatt ggagtacttc tgtgagtctg ccctccagct gactaaaacc       240
   ctgattgcta ggaaagaatg gaccacagtt gtctaagttc gag                         283
<210> 69
   <211> 365
   <212> DNA
   <213> Mus musculus
<400> 69
   aggcctacag acagttgtgc agtatagtgg gtgctgagaa tcaaagctgg tcctctagaa        60
   gagcagctag cttgttttct tcctttcttt ctttctttct ttctttcttt ctttctttct       120
   ttctttcttt ctttctttcc tgtaagtaca ctgtagctgt tttcagacac tccagaggag       180
   ggcgccagat ctcattacag atggttgtga gcaacagtgt ggttgctggg aattgaactc       240
   agaatcttca gaagagcaag ttagtgctct taaccactct gccatctctc cagcccccca       300
   tccccaacta gctagttttc ttgagaacat tatgactagc ccaatcacta gccctagcat       360
   ggaag                                                                   365
<210> 70
   <211> 140
   <212> DNA
   <213> Mus musculus
<400> 70
   agccacatgt gcttgctttc tttctttctt tctttctttc tttctttctt tctttctttc      60
   tttctttctt tctttctttc tttctttctt tctttcttta tcagaaacaa gagtatccat     120
   ttgcagcaat tgattaaggg                                                 140
<210> 71
   <211> 272
   <212> DNA
   <213> Mus musculus
<400> 71
   ctgagtgatg cagggtgcta ctctgcatct gtgccagaat tgcagaaggt tttctttctt      60
   tctttctttc tttctttctt tctttctttc tttctttctt tctttctttc tttctttctt     120
   tctttctttc ttttaactgt tgggagtgcc cacttggctt agtgattcaa aacaggtcct     180
   gaactacagg aaatcagctc ttccttgaat gagcttgcat agaaggagga ccctgatcct     240
   aactgataag aaaagcctct ggattcacag cc                                  272
<210> 72
   <211> 347
   <212> DNA
   <213> Mus musculus
<400> 72
   tgtagtcccc tggatcccat agtctagagt gacacataaa tatgaaaatg tttatgcaaa      60
   aatgcacaga ggagagaaag gaaacaatga gaaatagaaa gagagagaga tggagaaaga     120
   tagagacaga gagacaaata tagtgaaaga gagaaagaaa gaaagaaaga aagaaagaaa     180
   gaaagagaga aagaaagaaa gagcacaata ggatgcagaa gaaatggatt gagcaatatt     240
   gtctcagagt ataaggaaca aagaacagga tgaaggataa tgacccaaga gacaaaagac     300
   ttatcaattc accacactga atggtcaaaa gacaacttga gctggca                   347
<210> 73
   <211> 241
   <212> DNA
   <213> Rattus norvegicus
<400> 73
   gtcagaaggg gcagactcta aaaccttcaa agctatgcac tgtagacttt tgaatactgc        60
   tttctaagga catcttaaca gaaatgcggt ttgtttattg aatttattct ttccttcctt       120
   ccttctttct ttctttcttt ctttctttct ttctttcttt ctttctttct ttctttcttt       180
   ctttctttct ttctttttat actccagatt taattccctt ccagttcacc atcccaagtg       240
   a                                                                       241
<210> 74
   <211> 246
   <212> DNA
   <213> Rattus norvegicus
<400> 74
   tcacttggga tggtgaactg gaagggaatt aaatctggag tataaaaaga aagaaagaaa        60
   gaaagaaaga aagaaagaaa gaaagaaaga aagaaagaaa gaaagaaaga aagaaagaag       120
   gaaggaagga aggaaagaat aaattcaata aacaaaccgc atttctgtta agatgtcctt       180
   agaaagcagt attcaaaagt ctacagtgca tagctttgaa ggttttagag tctgcccctt       240
   ctgact                                                                  246
<210> 75
   <211> 230
   <212> DNA
   <213> Rattus norvegicus
<400> 75
   cagacctcag ggcatttcca tattctaagt tagcaactat acaagttatg aggactagta        60
   caaaatgccc agcatctatg tatctatgta tctatgtatc tatgtatcta tgtatctatg       120
   tatctatgta tcttctatct atctatctat ctatctatct atctatctat ctatctatct       180
   atctatctat ctatcaatca tctaagaaac cagagaacaa agagccatat                  230
<210> 76
   <211> 176
   <212> DNA
   <213> Rattus norvegicus
<400> 76
   cagatctcag ggcattccca tattctaagt caacaactct tcatgttatg aagactagta        60
   tgaaatcccc agcatctatc tatctatcta tctatctatc tatctatcta tctatctatc       120
   tatctatcta tctatctatc gatctatcta agaaaccaga aaacaaagag ccatat           176

## Beschreibung

### Gebiet der Erfindung

Die vorliegende Erfindung betrifft allgemein die Analyse genetischer Marker bei Individuen nicht-humanen Ursprungs, insbesondere auf die Genotypisierung von Auszuchtpopulationen von Nagern oder Fischen. Die Erfindung betrifft speziell die simultane Amplifikation von mindestens 5 verschiedenen polymorphen autosomalen Markern und mindestens zwei polymorphen Y-chromosomalen Markern der Maus in einem Reaktionsansatz mit Hilfe der Polymerase-Kettenreaktion oder anderer Multiplexverfahren und dem Nachweis der spezifischen Allele für jeden Marker des Multiplexverfahrens. Die Erfindung betrifft weiterhin ein Kit zur genetischen Identifizierung und/oder zur Unterscheidung von zwei oder mehr Tieren aus DNA-Extrakten von Individuen aus Wildtyp-Populationen, aus unterschiedlichen Inzucht- oder Auszuchtstämmen, des identischen Inzucht- oder Auszuchtstammes oder von Substämmen des identischen Inzucht- oder Auszuchtstammes, insbesondere einer Ratte oder Maus.

### Stand der Technik:

Tierexperimente sind nach wie vor ein unabdingbarer Bestandteil der modernen Forschung. Mit dem Ziel der Gewährleistung reproduzierbarer Experimente ist neben der Standardisierung von Zuchtbedingungen in der Tierhaltung auch die Standardisierung der genetischen Qualität der verwendeten Versuchstiere unabdingbar. Für den Züchter solcher Tiere bedeutet das den Einsatz von definierten Systemen zur Stabilisierung genetischer Strukturen. Solche Strukturen sollen eine Standardisierung über den Zeitraum von vielen Generationen sowie über die geografische Distanz gewährleisten. Letzteres bezieht sich auch auf die Bereitstellung von Versuchsstämmen mit identischer Bezeichnung aus unterschiedlichen Zuchtstandorten bzw. von unterschiedlichen Züchtern.

Bei Inzuchtstämmen handelt es sich um Modelle bei der Maus und Ratte, bei denen aufgrund einer konsequenten Bruder-Schwester-Verpaarung über viele Generationen hinweg die genetische Vielfalt zwischen den Individuen verloren gegangen und somit vernachlässigbar ist. Tiere eines Inzuchtstammes oder Substammes eines Inzuchtstammes sind zu über 99,9% genetisch identisch und zudem homozygot. Experimente mit solchen Tieren ermöglichen die statistische Absicherung von experimentellen Daten, da sie gleichzeitig an einer Vielzahl von quasi eineiigen Zwillingen erhoben werden können. Für die Übertragung experimenteller Erkenntnisse auf den Menschen haben solche Tiermodelle jedoch nur eine begrenzte Aussagekraft, da hier künstliche (Inzucht)Populationen abgebildet werden, die so in der Natur nicht vorkommen.

Parallel zu Inzuchtstämmen ergibt sich gleichfalls die Notwendigkeit der Verfügbarkeit von standardisierten Stämmen, bei denen die genetische Vielfalt erhalten wurde. Beispiele für solche Stämme sind Mosaik- und Auszuchtpopulationen. Sie dienen als praktisches Modell für nicht ingezüchtete Populationen, wie sie typischerweise auch bei Tier und Mensch vorkommen. Mit Hilfe von Auszuchtpopulationen soll die genetische Variation innerhalb einer Population abgebildet und in entsprechenden Experimenten genutzt werden. Der Genotyp eines jeden Auszuchttieres innerhalb einer Auszuchtpopulation ist einmalig und wiederholt sich bei keinem zweiten Tier. Ziel von Auszuchtpopulationen ist es, innerhalb einer geschlossenen, genetisch gut charakterisierten Population im Verlauf der Generationen eine definierte, möglichst hohe genetische Heterogenität unter Einhaltung bestimmter Variationsgrenzen zu bewahren. Solche Auszuchtstämme gibt es für Maus, Ratte und Zebrafisch.

Eine hohe genetische Variabilität kann z.B. erreicht werden, indem während der Aufbauphase einer Auszuchtpopulation verschiedene, wenig verwandte Stämme oder Inzuchtstämme eingekreuzt werden. Anschließend wird die Population geschlossen, es sind dann nur noch Verpaarungen innerhalb der Population erlaubt. Im Gegensatz zu Inzuchtstämmen werden keine miteinander verwandten Elterntiere miteinander verpaart. Damit soll eine stabile und standardisierte genetische Variabilität sichergestellt werden. Die wesentlichen Anforderungen an ein Auszuchtsystem wurden von Rapp (1972) zusammengefasst: (1) maximaler Erhalt populationsspezifischer Allel- und Genotypfrequenzen, (2) Minimierung der Zunahme der Homozygotie und des Inzuchtgrades, (3) Vermeidung von Sublinienentstehung, (4) einfache Anwendbarkeit. Streng genommen handelt es sich um eine genetisch gut charakterisierte, jedoch begrenzte Population von Tieren, die sich aus Individuen zusammensetzt, die ursprünglich von unterschiedlichen Stämmen stammen. Daher ist die fachlich korrekte Bezeichnung "Auszuchtpopulation". Ausgehend von der Bezeichnung "Inzuchtstämme" und zur Verdeutlichung des diametralen Unterschiedes zwischen Auszucht- und Inzuchtprinzip, wird in der Literatur häufig auch die Bezeichnung "Auszuchtstamm" genutzt, was somit als Synonym für "Auszuchtpopulationen" steht.

Für die zuverlässige Reproduzierbarkeit von Versuchsergebnissen mit Auszuchttieren müsste für jedes Experiment ein repräsentativer Querschnitt der Genotypenverteilung der konkreten Auszuchtpopulation vorliegen. Sofern keine genetischen Marker zur Beschreibung der genetischen Vielfalt eingesetzt werden, stellt das für den Züchter eine große Herausforderung dar. Für den Anwender ist die Situation noch komplizierter. Häufig werden für die einzelnen Versuchsabschnitte nur sehr begrenzte Tierzahlen beim Züchter bestellt und es ist keinerlei Kontrolle darüber möglich, ob die einzelnen Tiere einer einzigen (z. B. ein Elternpaar) oder unterschiedlichen Zuchtgruppen der Auszuchtpopulation entstammen.

Bei der ursprünglichen Erstellung und Strukturierung von Auszuchtpopulationen werden unterschiedliche männliche Gründertiere (Foundertiere) eingekreuzt, die ihrerseits jeweils ihre individuelle Variante des Y-Chromosoms in die Auszuchtpopulation einbringen. Wird die Auszucht korrekt betrieben, so verbleiben alle ursprünglich vorhandenen Varianten in der Population. Bei unterschiedlichen Auszuchtpopulationen und auch innerhalb einer definierten Auszuchtpopulation dürfte eine Vielzahl an unterschiedlichen Y-Chromosomen vorliegen, die auch erhebliche genetische Unterschiede aufweisen können. Obwohl ein wesentlicher Aspekt zur Definition der genetischen Vielfalt einer Auszuchtpopulation, sind für die Anzahl an unterschiedlichen Y-Chromosomen bislang keinerlei wissenschaftliche Daten verfügbar.

Bei den gegenwärtig in der Forschung genutzten Inzuchtstämmen geht das Y-Chromosom auf einen der die beiden Typen Mus musculus musculus (M. m. musculus) bzw. Mus musculus domesticus (M. m. domesticus) zurück, die sich vor ca. 900.000 Jahren voneinander abgespalten haben. Genetische Analysen haben ergeben, dass die Mausspezies M. m. musculus, M. m. castaneus und M. m. molossinus alle ein Y-Chromosom vom Typ M. m. musculus tragen. M. m. domesticus dagegen stellt einen gesonderten, zweiten Grundtyp dar (Pertile et.al, 2009).

Es ist bekannt, dass das Y-Chromosom bei der Maus als globaler Regulator der Genomweiten Expression fungiert und das Einkreuzen eines Stamm-fremden Y-Chromosoms oft zu erheblichen Änderungen des Phänotyps bei männlichen und weiblichen Nachkommen führt (Nelson et al, 2010). Daher stellen unterschiedliche Varianten des Y-Chromosoms einen entscheidenden Parameter der genetischen und phänotypischen Varianz einer Auszuchtpopulation dar. Die Beschreibung der genetischen Vielfalt bedarf somit auch der Kenntnis über die Anzahl und die Art an unterschiedlichen Varianten des Y-Chromosoms in der Population. Bislang sind für Auszuchtstämme bei der Maus weder die Anzahl an verschiedenen Y-chromosomalen Haplotypen noch deren Zugehörigkeit zu M. m. musculus oder M. m. domesticus bekannt.

Während für Inzuchten bei der Maus ein großes Markerrepertoire an DNA-Polymorphismen genutzt wird, gibt es für die Auszuchten der Maus bislang keine standardisierten Monitoringprogramme bzw. Markersets (Kluge und Wedekind, 2016). Yalcin et al (2010) beschreiben die parallele Nutzung von mehreren 100.000 SNPs zur genetischen Charakterisierung von Auszuchtpopulationen der Maus.

Mikrosatelliten, auch Short tandem repeats (STRs) genannt, werden zur Genotypisierung von Inzuchtstämmen eingesetzt. Es handelt sich um kurze DNA-Sequenzen von 1 bis 6 Basen Länge, deren Grundmotiv sich wie Perlen aneinander gereiht mehrfach wiederholt, z.B. [CA]n, [GAC]n oder [GATA]n. Durch die unterschiedliche Anzahl an solchen Wiederholungen bei verschiedenen Individuen lassen sich solche voneinander unterscheiden. Witmer et al (2003) beschreiben die Nutzung von STRs mit einer 2er Wiederholungseinheit zur Unterscheidung der verschiedenen Inzuchtstämme der Maus. In US 2014/0066322 wird die Anwendung eines PCR-Multiplex zur Unterscheidung von Zelllinien bei der Maus beschrieben, bei dem 9 verschiedene STRs der Maus mit 2 weiteren humanen STR-Markern kombiniert werden. In WO 2016/008894 wird die Genotypisierung zur Unterscheidung einzelner Individuen innerhalb von Inzuchtstämmen beschrieben. In beiden Anmeldungen kommen STR-Marker zum Einsatz, deren Wiederholungseinheit aus 4 Nukleotiden besteht.

Alle verfügbaren Daten für STRs, die für die Maus erhoben wurden, basieren auf der Analyse von Inzuchtstämmen. Die Nutzung von STRs für Auszuchtpopulationen der Maus ist nicht bekannt. Biostatistische Parameter zur Bewertung der Qualität einzelner STR-Marker der Maus, wie Polymorphism information content (PIC) oder Heterozygotierate sind nicht verfügbar. Shang et al (2014) beschreiben 6 STRs, die zur Genotypisierung bei der Ratte für die Auszuchtstämme Wistar und Sprague Dawley genutzt wurden.

Die genetische Kontrolle von Auszuchtpopulationen bei der Maus, Ratte oder Zebrafisch ist daher ein Bereich, der noch deutlich zu optimieren ist (Kluge und Wedekind, 2016). Hierfür werden aussagefähige biostatistische Parameter benötigt, die bislang nicht verfügbar sind. Es wäre vorteilhaft, wenn eine Methode auf der Basis von autosomalen und geschlechtsspezifischen STR-Markern zur Verfügung stünde, mit deren Hilfe die Genotypisierung und umfassende biostatistische Charakterisierung von Auszuchtpopulationen ermöglicht wird. Eine geeignete Methode ist die Multiplex-STR-Analyse von polymorphen Tetranucleotid-STR Loci der Maus, was Gegenstand der aktuellen Erfindung ist.

### Zusammenfassung der Erfindung

Die Aufgabe der Erfindung besteht daher darin, die Nachteile des Standes der Technik zu überwinden und dem Züchter und Anwender verlässliche Mittel und Methoden zur Genotypisierung von Auszuchtpopulationen zur Verfügung zu stellen.

Diese Aufgabe wird gelöst durch Bereitstellung von besonders geeigneten STR-Markern, mit deren Hilfe dem Züchter und Anwender ein Set an Markern zur Genotypisierung von Auszuchtpopulationen zur Verfügung gestellt werden kann.

Die Aufgabe der Erfindung besteht weiterhin darin, dem Züchter zu ermöglichen, Auszuchtpopulationen genetisch zu charakterisieren, die genetische Vielfalt zu dokumentieren und ein genetisches Monitoring wichtiger biostatistischer Parameter während der Zucht durchzuführen. Aufgabe der Erfindung ist es weiterhin, anhand von autosomalen und Y-chromosomalen Markern eine Methode zur Bewertung der genetischen Vielfalt innerhalb einer Auszuchtpopulation zur Verfügung zu stellen. Aufgabe der Erfindung ist ebenfalls, durch die Anwendung eines einheitlichen Sets an Markern eine vergleichende Bewertung der Qualität von Auszuchtpopulationen untereinander zu ermöglichen. Weiterhin soll dem Anwender ermöglicht werden, die genetische Vielfalt der von kommerziellen Züchtern erworbenen Auszuchttiere unabhängig bewerten zu können.

Zur Lösung der vorstehend genannten Aufgaben stellt die Erfindung in einem Aspekt eine Methode zur genetischen Identifizierung und/oder zur Unterscheidung von zwei oder mehr Tieren aus unterschiedlichen Auszuchtstämmen, des identischen Auszuchtstammes oder von Substämmen des identischen Auszuchtstammes der Spezies Maus, Ratte, Hamster und Zebrafisch, bereit, wobei die Methode die simultane Amplifikation von mindestens 6, 7 oder 8, besonders bevorzugt mindestens 9 oder 10, am meisten bevorzugt mindestens 11 verschiedenen polymorphen autosomalen Markern und mindestens zwei polymorphen Y-chromosomalen Markern der jeweiligen Spezies in einem Reaktionsansatz mit Hilfe der Polymerase-Kettenreaktion oder anderer Multiplexverfahren und optional den Nachweis der spezifischen Allele für jeden Marker des Multiplexverfahrens umfasst.

Die Methode umfasst ebenfalls die Bereitstellung von entsprechenden Oligonukleotid-Primerpärchen, wobei jedem Marker ein Primerpärchen zugeordnet wird, welches beiderseits der Markerregion spezifisch hybridisiert. Die Methode beschreibt weiterhin die simultane Amplifikation von mindestens 7, vorzugsweise mindestens 8, 9 oder 10, besonders bevorzugt mindestens 11 oder 12, am meisten bevorzugt mindestens 13 der ausgewählten Marker in einem Multiplex-Reaktionsansatz und die Entstehung eines Gemisches von Allelen mit Reaktionsprodukten für jeden der beteiligten Marker.

Die Methode umfasst weiterhin die Detektion der einzelnen Allele im Allelgemisch und deren eindeutige Zuordnung zu einem Marker sowie zu einem definierten Allel für den konkreten Marker.

In einer gesonderten Ausführungsform der vorliegenden Erfindung wird eine Methode bereitgestellt, welche die Zuordnung des **Y-Chromosoms** von Auszuchtpopulationen **zum Typ M. m. musculus** oder **M. m. domesticus** mit Hilfe von STR-Markern ermöglicht.

In einer weiteren Ausführungsform der vorliegenden Erfindung wird eine Methode bereitgestellt, welche die Identifizierung verschiedener Varianten des **Y-Chromosoms innerhalb einer Auszuchtpopulation** der Maus mit Hilfe von STR-Markern ermöglicht.

In einer gesonderten Ausführungsform der vorliegenden Erfindung wird eine Methode bereitgestellt, welche die Identifizierung verschiedener Varianten des **Y-Chromosoms der Ratte** mit Hilfe von STR-Markern ermöglicht.

Das erfindungsgemäße Markerset kann zusätzlich durch solche Marker ergänzt werden, die eine Zuordnung zu einzelnen Mausstämmen ermöglichen. Hierfür eignen sich beispielsweise bekannte Stamm-spezifische Insertions-Deletions-Polymorphismen (Indels) oder SNPs. Exemplarisch für solche Marker kann die von Clapcote and Roder (2006) beschriebene Deletion von 25 bp im Gen Disc1 für den Stamm 129 aufgeführt werden. Diese zusätzlichen Marker sind besonders geeignet für den Einsatz des erfindungsgemäßen STR-Multiplex-Assays bei Zuordnung von Foundertieren einer Auszuchtpopulation zu bekannten Inzuchtstämmen, der Charakterisierung von transgenen Linien der Maus sowie der Prüfung der Herkunft von Zelllinien.

Mit vorliegender Erfindung wird in einem weiteren Aspekt ein Kit zur Verfügung gestellt, mit dessen Hilfe die in einer DNA-Probe enthaltenen Allele eines Markersets nachgewiesen werden können. Das Kit enthält alle notwendigen Komponenten zur Durchführung einer simultanen Ko-Amplifikation und zum Nachweis von Allelen unter Einbeziehung von mindestens 6, 7 oder 8, besonders bevorzugt mindestens 9 oder 10, am meisten bevorzugt mindestens 11 verschiedenen autosomalen STR-Markern und mindestens zwei, vorzugsweise drei Y-chromosomalen STR-Markern für mindestens eine DNA-Probe.

Die nachfolgende detaillierte Beschreibung und die beigefügte Figur und Ausführungsbeispiele sollen das Wesen der Erfindung näher erläutern, weitere Anwendungsmöglichkeiten aufzeigen und die sich daraus ergebenden die Vorteile demonstrieren.

In einem weiteren Aspekt stellt die Erfindung ein Kit zur genetischen Identifizierung und/oder zur Unterscheidung von zwei oder mehr Tieren aus unterschiedlichen Stämmen, des identischen Stammes oder von Substämmen des identischen Stammes der der Spezies Maus, Ratte, Hamster und Zebrafisch aus DNA-Extrakten, wobei das Kit umfasst:
(a) ein Set von Oligonukleotid-Primerpaaren, bei denen jedes Primerpaar spezifisch an die flankierenden DNA-Abschnitte eines Locus aus dem Set der STR-Loci bindet und welches mindestens 6, 7 oder 8, besonders bevorzugt mindestens 9 oder 10, am meisten bevorzugt mindestens 11 autosomale STR-Marker umfasst, die gleichzeitig in einem Reaktionsansatz ko-amplifiziert werden können;
(b) ein Set von Oligonukleotid-Primerpaaren, die spezifisch an die flankierenden DNA-Abschnitte des Y-Chromosoms binden und welches mindestens zwei, vorzugsweise mindestens drei Y-chromosomale STR-Marker umfasst, die gleichzeitig mit den autosomalen STR-Loci (a) in einem Reaktionsansatz ko-amplifiziert werden können;
(c) Reagenzien, die für die Durchführung von mindestens einer Multiplex-PCR ausreichend sind; und
(d) einen Größenstandard, welcher unterschiedliche DNA-Marker mit einer bekannten Fragmentlänge enthält.

In einer bevorzugten Ausführungsform der Erfindung besteht das Kit aus den Bestandteilen (a) bis (d).

### Detaillierte Beschreibung der Erfindung

### Definitionen

**Allel:** Als Allel bezeichnet man verschiedene Zustandsformen eines Gens oder einer Gensequenz in einem definierten DNA-Bereich eines Chromosoms.

**Amplifikation:** Amplifikation bezeichnet die Vervielfältigung von DNA-Abschnitten. Diese kann natürlich vorkommen oder aber künstlich erzeugt werden *(in vitro).* Letzteres wird beispielsweise in der Molekularbiologie über das PCR-Verfahren realisiert.

**Haplotyp:** Haplotyp bezeichnet einen haploiden Genotyp, eine einmalige Variante einer Nukleotidsequenz auf ein und demselben Chromosom. Das Y-Chromosom kommt im Genom nur einfach vor und kann mit keinem anderen Chromosom DNA-Abschnitte austauschen, der Y-chromosomale Haplotyp bleibt somit konstant.

**Multiplex:** Im Gegensatz zu einer Singleplex-PCR, bei der lediglich ein Primerpaar genutzt wird, enthält ein Multiplex-PCR-Ansatz mehrere verschiedene Primerpaare, wobei jedes der Primerpaare eine unterschiedliche Region der chromosomalen DNA amplifiziert.

PCR: Die Polymerase-Kettenreaktion (engl. Polymerase chain reaction) ist eine Methode, mit der *in vitro* die DNA mit Hilfe von DNA-Polymerasen (z.B. Taq-Polymerase) vervielfältigt wird. Als Startpunkt für die Neusynthese werden spezifische Primer eingesetzt, die spezifisch an definierte DNA-Abschnitte binden.

**polymorph:** Ein Marker ist polymorph, wenn er mindestens zwei oder mehr unterschiedliche Allele aufweist.

**Primer** / **Oligonukleotid-Primer:** Als Primer wird in der Molekularbiologie ein Oligonukleotid bezeichnet, das als Startpunkt für die Amplifikation von DNA-Abschnitten dient.

### Bevorzugte Ausführungsformen der Erfindung

Besonders gute Ergebnisse wurden bei der Genotypisierung von Auszuchtpopulationen erzielt, wenn das Set von Loci für die DNA-Probe, aus denen die autosomalen STR-Marker ausgewählt werden, mindestens 11 autosomale STR-Marker umfasst, die gleichzeitig in einem Reaktionsansatz ko-amplifiziert werden können.

Gemäß einem ersten Aspekt stellt die Erfindung deshalb eine Methode zur genetischen Identifizierung und/oder zur Unterscheidung von zwei oder mehr Tieren aus unterschiedlichen Auszuchtstämmen, des identischen Auszuchtstammes oder von Substämmen des identischen Auszuchtstammes der Spezies Maus, Ratte, Hamster oder Zebrafisch bereit, umfassend folgende Teilschritte
(a) die Verwendung von mindestens einer DNA-Probe, die analysiert werden soll,
(b) die Auswahl eines Sets von Loci für die DNA-Probe, welche mindestens 11 autosomale STR-Marker umfasst, die gleichzeitig in einem Reaktionsansatz ko-amplifiziert werden können,
(c) die Auswahl eines Sets von Loci für die DNA-Probe, welche mindestens zwei Y-chromosomale STR-Marker umfasst, die gleichzeitig mit den autosomalen STR-Loci (b) in einem Reaktionsansatz ko-amplifiziert werden können,
(d) die Verwendung eines Sets von Oligonukleotid-Primerpaaren, bei denen jedes Primerpaar spezifisch an die flankierenden DNA-Abschnitte eines STR-Markers aus dem Set der STR-Loci bindet,
(e) die Ko-Amplifikation der für das Set ausgewählten Loci, wobei das Reaktionsprodukt ein Gemisch darstellt, bestehend aus amplifizierten Allelen von jedem der ko-amplifizierten STR-Loci des Sets, und
(f) die Analyse des Allelgemisches mit dem Ziel der Bestimmung und Zuordnung der spezifischen Allele für jeden der amplifizierten Loci des Sets für die gegebene DNA-Probe;
wobei die autosomalen STR-Loci durch einen hohen Wert für den "Polymorphism information content" charakterisiert werden, der im Bereich von 0,30 und 0,99 liegt, vorzugsweise im Bereich von 0,50 bis 0,99, vorzugsweise im Bereich zwischen 0,60 und 0,99.

In einer bevorzugten Ausführungsform der Erfindung besteht die Methode zur genetischen Identifizierung und/oder zur Unterscheidung von zwei oder mehr Tieren aus unterschiedlichen Wildtyp-Populationen, aus unterschiedlichen Auszuchtstämmen, des identischen Auszuchtstammes oder von Substämmen des identischen Auszuchtstammes der Spezies Maus, Ratte, Hamster und Zebrafisch aus den Teilschritten (a) bis (f).

In einer weiteren bevorzugten Ausführungsform der erfindungsgemäßen Methode stammt die DNA-Probe von mindestens einem Tier der Spezies Maus, Ratte, Hamster oder Zebrafisch oder von einer Zelllinie der Spezies Maus, Ratte, Hamster oder Zebrafisch.

Die Anwendung der erfindungsgemäßen Methode ist keineswegs nur auf Auszuchtstämme begrenzt. Sie kann in allen Fällen genutzt werden, bei denen eine vergleichende DNA-Analyse genetischer Marker sinnvoll ist, wie beispielsweise für die Authentifizierung von Zelllinien, die genetische Charakterisierung von Wildfängen (Wildtyp-Populationen), Inzuchtstämmen sowie von genetisch modifizierten Linien der Maus. Beispielsweise kann ein Multiplex, der jeweils einen Marker pro Chromosom enthält, für die Überwachung des Ploidiegrads von Zelllinien und zur Bewertung der Notwendigkeit der Durchführung einer Karyotypisierung einer Zelllinie genutzt werden.

Die Vorliegende Erfindung bezieht sich auf das bereits eingehend erläuterte Erfordernis der Kontrolle der genetischen Qualität von Auszuchtpopulationen. Hierfür werden aussagefähige biostatistische Parameter benötigt, die im Ergebnis einer Genotypisierung sowohl vom Züchter als auch vom Anwender erhoben werden können. Ein besonderer Vorteil der Erfindung besteht darin, dass der erfindungsgemäße Multiplex-Assay auf der Grundlage von STR-Markern mit tetranukleotiden Wiederholungseinheiten genutzt werden kann, um beliebige Auszuchtstämme der Maus, Ratte, Hamster oder des Zebrafischs, vorzugsweise der Maus oder Ratte, besonders bevorzugt der Maus, in einem ausreichenden Maß genetisch zu charakterisieren, untereinander zu vergleichen. Dadurch wird das genetische Monitoring von Auszuchtpopulationen bei der Maus, Ratte, Hamster oder dem Zebrafisch, vorzugsweise der Maus oder Ratte, besonders bevorzugt der Maus, erheblich vereinfacht.

In einer Ausführungsform stellt die vorliegende Erfindung eine Methode bereit, welche die Zuordnung von Allelen definierter STR-Marker in einer DNA-Probe ermöglicht. Diese Methode umfasst in einer bevorzugten Ausführungsform die Auswahl einer für die Analyse bestimmten DNA-Probe und die Auswahl von mindestens 11 autosomalen STR-Markern aus einer Gruppe von polymorphen STR-Markern der Maus: D1Mmu121, D2Mmu008, D3Mmu158, D4Mmu155, D5Mmu108, D6Mmu120, D7Mmu003, D8Mmu127, D9Mmu100, D10Mmu043, D11Mmu030, D12Mmu056, D13Mmu096, D14Mmu074, D15Mmu084, D16Mmu030, D17Mmu041, D18Mmu069, D19Mmu008. Die einzelnen autosomalen Marker sind erfindungsgemäß auf unterschiedlichen Chromosomen lokalisiert, so dass sie untereinander genetisch nicht gekoppelt sind. Das hat den Vorteil, dass damit die Anwendung einfacher biostatistischer Parameter zur Beschreibung einer Population ermöglicht wird.

In einer bevorzugten Ausführungsform der erfindungsgemäßen Methode werden in Schritt (b) mindestens zwei der elf autosomalen STR-Loci aus einer Gruppe von Loci, ausgewählt, wobei die Gruppe die Loci D1Mmu121, D2Mmu008, D3Mmu158, D4Mmu155, D5Mmu108, D6Mmu120, D7Mmu003, D8Mmu127, D9Mmu100, D10Mmu043, D11Mmu030, D12Mmu056, D13Mmu096, D14Mmu074, D15Mmu084, D16Mmu030, D17Mmu041, D18Mmu069 und D19Mmu008 umfasst.

In einer bevorzugten Ausführungsform der erfindungsgemäßen Methode besteht in Schritt (b) das Set aus elf autosomalen Loci aus den Loci D2Mmu008, D3Mmu158, D4Mmu155, D6Mmu120, D7Mmu003, D8Mmu127, D10Mmu043, D13Mmu096, D14Mmu074, D16Mmu030 und D18Mmu069.

Weiterhin umfasst die erfindungsgemäße Methode die Auswahl von mindestens zwei auf dem Y-Chromosom lokalisierten STR-Markern aus einer Gruppe von möglichen STR-Markern der Maus: DYMmu001, DYMmu002 und DYMmu003. Der Marker DYM003 wird genutzt, um eine Zuordnung zum Typ M. m. musculus oder M. m. domesticus zu ermöglichen. Mindestens ein weiterer polymorpher STR-Marker von DYMmu001 oder DYMmu002 wird zur Unterscheidung unterschiedlicher Haplotypen eingesetzt.

Vorzugsweise befindet sich das Set von Loci in Schritt (c) der erfindungsgemäßen Methode auf dem Y-Chromosom und kann in vorteilhafter Weise für die Identifizierung des Geschlechts in der DNA-Probe des Individuums in Schritt (a) genutzt werden. Ein weiterer Vorteil des Sets von Loci gemäß Schritt (c) besteht darin, dass dieses Set von Loci zur Identifizierung des Geschlechts zwischen den Y-Chromosomen vom Typ Mus musculus musculus und vom Typ Mus musculus domesticus unterscheiden kann.

In einer weiteren bevorzugten Ausführungsform stellt der Locus zur Bestimmung des Geschlechts einen polymorphen STR-Locus der Maus dar und ist ausgewählt aus der Gruppe umfassend die Loci DYMmu001, DYMmu002 und DYMmu003. Ein solcher Locus hat den Vorteil, dass er Y-chromosomale Haplotypen der Maus unterscheiden kann.

In einer weiteren bevorzugten Ausführungsform stellt der Locus zur Bestimmung des Geschlechts einen polymorphen STR-Locus der Ratte dar und ist ausgewählt aus der Gruppe umfassend die Loci DYRno004, DYRno165 und DYRno304. Ein solcher Locus hat den Vorteil, dass er Y-chromosomale Haplotypen der Ratte unterscheiden kann.

Damit ist es möglich, innerhalb einer konkreten Auszuchtpopulation eine Zuordnung von männlichen Individuen zu unterschiedlichen Haplotypen, und ebenso die Zuordnung zu einem der beiden Grundtypen des Y-Chromosoms M. m. musculus oder M. m. domesticus vorzunehmen. Dieser Ansatz zur Beschreibung der genetischen Vielfalt von Auszuchtpopulationen über das Y-Chromosom wird erstmals durch die vorliegende Erfindung zur Verfügung gestellt und kann zukünftig zum genetischen Monitoring eingesetzt werden.

In einer besonders bevorzugten Ausführungsform wird in Schritt (e) der erfindungsgemäßen Methode eine Multiplex-Amplifikation mit mindestens dreizehn Paaren an Oligonukleotid-Primern durchgeführt, welche mindestens elf autosomale und zwei Y-chromosomale STR-Loci umfasst.

Die amplifizierten Allele werden vorzugsweise vor der Auswertung in Schritt (f) mittels eines analytischen oder semipräparativen Verfahrens separiert. Ein bevorzugtes Separationsverfahren ist die Gelelektrophorese. Besonders bevorzugt ist die Polyacrylamid-Gelelektrophorese oder die Kapillar-Gelelektrophorese.

Es hat sich weiterhin als vorteilhaft erwiesen, wenn die Primerpaare für das nachfolgende Nachweisverfahren markiert werden. In einer bevorzugten Ausführungsform des erfindungsgemäßen Verfahrens wird deshalb mindestens ein Oligonukleotid-Primer eines Primerpaares kovalent mit einem Detektionsfarbstoff gekoppelt, vorzugsweise mit einem Fluoreszenzfarbstoff.

Besonders bevorzugt ist es, wenn mindestens vier verschiedene Farbstoff-gekoppelte Primer mit vier verschiedenen Fluoreszenzfarbstoffen eingesetzt werden. Dies vereinfacht die Analyse des Allelgemisches in Schritt (f), in dem die spezifischen Allele für jeden der amplifizierten Loci des Sets für die gegebene DNA-Probe bestimmt und zugeordnet werden. Es ist damit ein direkter Nachweis der Allele möglich.

Die Fluoreszenzfarbstoffe können z.B. ausgewählt sein aus den Gruppen der Phycobiline, Rhodamine und Safranine. Geeignete Fluoreszenzfarbstoffe können z.B. ausgewählt sein aus der Gruppe, die aus Acridingelb, Acridinorange, Aequorin, Aesculin, Allophycocyanin, 7-Aminoactinomycin, ATTO-Farbstoffe, Auramin O, Berberin, 9,10-Bis(phenylethinyl)anthracen, Calcein, 6-Carboxyfluorescein, Chinin, Cumarin-Farbstoffe, Cyaninen, 4',6-Diamidin-2-phenylindol, 2,7-Dichlorfluorescein, 9,10-Diphenylanthracen, Eosin B, Eosin Y, Epicocconon, Ethidiumbromid, 6-FAM-phosphoramidit, Flavinen, Fluoren, Fluorescein, Fluorescein Arsen Helix Bindern, Fura-2, Furaptra, Grün fluoreszierendes Protein (GFP), Hoechst 33342, IAEDANS, Indischgelb, Indocyaningrün, Luciferinen, Merbromin, N-Methylacridon, Nilblau, Nilrot, Phycocyanin, Phycoerythrin, Propidiumiodid, Pyranin, Rhodamin B, Rubren, Safranin T, Stilben, SYBR Green I, SYPRO Orange, SYPRO Red, SYPRO Ruby, Texas Red, TMRM+, Umbelliferon und Xylenolorange besteht.

Die Fluoreszenzfarbstoffe sind vorzugsweise kovalent an die Nukleinsäuren in der Probe gebunden.

Letztlich ist es von Vorteil, wenn die Zuordnung der amplifizierten Allele in Schritt (f) anhand des Vergleiches mit einem Größenstandard erfolgt. In einer weiteren bevorzugten Ausführungsform des erfindungsgemäßen Verfahrens erfolgt deshalb die Zuordnung der amplifizierten Allele in Schritt (f) anhand des Vergleiches mit einem Größenstandard, wobei der Größenstandard ein Gemisch aus DNA-Fragmenten mit bekannter Größe und/oder ein Locus-spezifisches Gemisch bekannter Allele darstellt.

In einem weiteren Aspekt stellt die Erfindung ein Kit zur genetischen Identifizierung und/oder zur Unterscheidung von zwei oder mehr Tieren aus unterschiedlichen Stämmen, des identischen Stammes oder von Substämmen des identischen Stammes aus DNA-Extrakten bereit, umfassend:
(a) ein Set von Oligonukleotid-Primerpaaren, bei denen jedes Primerpaar spezifisch an die flankierenden DNA-Abschnitte eines Locus aus dem Set der STR-Loci bindet und welches mindestens 11 autosomale STR-Marker umfasst, die gleichzeitig in einem Reaktionsansatz ko-amplifiziert werden können, bestehend aus den Loci: D2Mmu008, D3Mmu158, D4Mmu155, D6Mmu120, D7Mmu003, D8Mmu127, D10Mmu043, D13Mmu096, D14Mmu074, D16Mmu030, D18Mmu069
(b) ein Set von Oligonukleotid-Primerpaaren, die spezifisch an die flankierenden DNA-Abschnitte des Y-Chromosoms binden und welches mindestens drei Y-chromosomale STR-Marker umfasst, die gleichzeitig mit den autosomalen STR-Loci (a) in einem Reaktionsansatz ko-amplifiziert werden können, bestehend aus den Loci DYMmu001, DYMmu002 und DYMmu003
(c) Reagenzien, die für die Durchführung von mindestens einer Multiplex-PCR ausreichend sind
(d) einen Größenstandard, welcher unterschiedliche DNA-Marker mit einer bekannten Fragmentlänge enthält;
wobei es sich bei den Tieren vorzugsweise um Stämme der Maus handelt.

In einer besonders bevorzugten Ausführungsform umfasst das Kit weiterhin
(e) einem Größenstandard, welcher ein Locus-spezifisches Gemisch bekannter Allele enthält.

In einer weiteren bevorzugten Ausführungsform der Erfindung besteht das Kit zur genetischen Identifizierung und/oder zur Unterscheidung von zwei oder mehr Tieren aus unterschiedlichen Stämmen, des identischen Stammes oder von Substämmen des identischen Stammes der Maus aus DNA-Extrakten aus den Bestandteilen a. bis d.

Die vorliegende Erfindung stellt somit eine Methode und ein Kit zur Genotypisierung von Individuen bei Tierarten, die in Form von Auszuchtpopulationen gezüchtet werden, speziell von Auszuchtpopulationen bei der Maus oder Ratte, bereit. Sie ermöglicht die Genotypisierung einzelner Individuen, die Identifizierung unterschiedlicher Haplotypen des Y-Chromosoms und die Beschreibung der genetischen Vielfalt einer konkreten Auszuchtpopulation. Die hierbei offen gelegte Methode und das Kit ermöglichen die simultane Analyse genomischer DNA-Abschnitte bei vorzugsweise der Maus mit Hilfe von STR-Markern auf der Basis von Tetranukleotid-Wiederholungseinheiten, wobei mindestens elf verschiedene autosomale STR Marker und mindestens zwei weitere STR-Marker für das Y-Chromosom in einem Reaktionsansatz ko-amplifiziert werden.

### Allgemeine Methodenbeschreibung

Regionen mit potenziellen STR-Markern wurden mit Hilfe der Software FastPCR (PrimerDigital Ltd, Kalendar et al. 2014) identifiziert, indem die in GeneBank hinterlegten Fasta-Files der Chromosomen der Maus (Version GRCm38.p4) und Ratte (Version Rnor_6.0) in die Software importiert wurden. Die Identifizierung von Regionen mit potenziellen STR-Markern erfolgte mit dem Modul "SSR Search". Auswahlkriterium war das Vorliegen einer Tetranukleotid-Wiederholungseinheit, die mindestens 10fach tandemartig wiederholt vorliegen musste. Mittels PrimerBLAST wurden Primer generiert, die für die Analyse der Marker im Einzelansatz geeignet sind.

Genomische DNA wurde aus Biopsien der Schwanzspitze oder Ohrlochstanzen mit Hilfe des NucleoSpin Tissue Kit (Macherey-Nagel, Düren, Germany) entsprechend den Vorgaben des Herstellers extrahiert.

Auf der Grundlage der Analyse von 150 Individuen aus verschiedenen Inzuchtstämmen und Auszuchtpopulationen sowie von Substämmen von Inzuchtstämmen der Maus wurden für 240 verschiedene STR-Marker die in den DNA-Proben nachweisbaren Allele bestimmt. Nicht alle Kandidaten-Marker führten zu amplifizierbaren DNA-Produkten in allen getesteten Stämmen. Daher war ein weiteres Kriterium zur Auswahl von geeigneten Markern, dass sie in allen Auszuchtpopulationen und in allen Inzuchtstämmen eingesetzt werden können.

Die Eignung der STR-Marker zur Beschreibung der genetischen Vielfalt von Auszuchtpopulationen wurde anhand der genetischen Analyse von insgesamt 70 verschiedenen männlichen Individuen der Auszuchtstämme CD1, SWISS und NMRI über die Bestimmung der biostatistischen Parameter Polymorphism information content (PIC) und Heterozygotenrate (Het) geprüft. Für jedes Chromosom wurde derjenige STR-Marker ausgewählt, der den höchsten Wert für PIC aufweist.

Für alle STR-Marker wurden Oligonukleotid-Primer identifiziert, die zur simultanen Amplifikation von mindestens 11 verschiedenen autosomalen STR-Markern geeignet sind. Überraschend waren viele der mit PrimerBLAST generierten Primer nicht optimal für Multiplex-Anwendungen geeignet. Die anschließende Optimierung der Oligonukleotid-Sequenzen im Hinblick auf Multiplexfähigkeit erfolgte manuell ohne Software indem eine Vielzahl an unterschiedlichen Primern getestet wurde. Alle Primer werden bei einer Annealingtemperatur von 58°C eingesetzt. Forward-Primer wurden mit einem der Fluoreszenzfarbstoffe 6FAM^{™} (blau), DY530^{™} (grün), DY510XL^{™} (rot), ATTO550^{™} (gelb) gekoppelt (Eurogentec, Niederlande). Die Primersynthese erfolgte durch biomers (biomers.net GmbH, Ulm, Germany) und Eurogentec (Eurogentec GmbH, Köln, Germany).

**Tabelle** 1 enthält die Auflistung der erfindungsgemäßen autosomalen und Y-chromosomalen STR-Marker der Maus und die für die Amplifikation im Multiplex-Verfahren eingesetzten Oligonukleotid-Primer. Für jeden Primer ist dessen chromosomale Lokalisation in Basenpaaren angegeben, die auf der Basis der Referenz-DNA (NCBI, Version GRCm38.p4) ermittelt wurde. Die Bezeichnung der STR-Marker hat das Ziel, bereits anhand des Namens wesentlichen Kenndaten zu erfassen: "D" steht für DNA, die Benennung des Chromosoms (Angabe direkt nach dem "D", 1 bis 19 für die Autosomen bzw. X und Y für die Geschlechtschromosomen), Identifizierung der Herkunft der DNA (z. B. Mmu - Mus musculus, Rno - Rattus norwegicus, Dre - Danius rerio) sowie die ungefähre Position auf dem Chromosom (in Megabasen). Beim STR-Marker D1Mmu121 handelt es sich beispielsweise um einen DNA-Marker der der Maus, der sich auf Chromosom 1 im Bereich von ca. 121 Megabasen befindet.

Weiterhin ist in der Tabelle 1 für jeden Marker die Struktur der Wiederholungseinheit gezeigt. Die angegebene Anzahl an Wiederholungseinheiten bezieht sich auf die in der Datenbank des NCBI hinterlegte Referenzsequenz für den Stamm C57BL/6J. Die Benennung der Allele erfolgte entsprechend den Richtlinien zur Standardisierung der Nomenklatur für STR-Marker in der Forensik (DNA Recommendations, 1997).

Die zu einem Marker zugehörigen Primer werden mit einer Konzentration von 5 pmol/µl miteinander im Verhältnis von 1 : 1 gemischt. Die PCR wurde mittels Snooplex^{®} FastPrep PCR Reagenzien (GVG Genetic Monitoring, Leipzig) in einem Gesamtvolumen von 25 µl durchgeführt. Der Reaktionsansatz für die PCR-Analyse von STR-Markern im Einzelansatz bestand aus folgenden Komponenten: 16,2 µl Nuklease-freies Wasser, 5 µl 5x PCR-Puffer, 1,0 µl Primergemisch, 0,8 µl Taq DNA Polymerase und 2 µl DNA. Beim erfindungsgemäßen Multiplex-PCR-Ansatz wurden Primergemische mit folgenden Mengen eingesetzt: D2Mmu008 (0,8 µl), D3Mmu158 (1,2 µl), D4Mmu155 (0,8 µl), D6Mmu120 (1,6 µl), D7Mmu003 (1,2 µl), D8Mmu127 (1,2 µl), D10Mmu043 (0,4 µl), D13Mmu096 (0,7 µl), D14Mmu074 (1,6 µl), D16Mmu030 (1,6 µl), D18Mmu069 (1,2 µl), DYMmu001 (1,0 µl), DYMmu002 (0,8 µl), DYMmu003 (1,0 µl). Das Auffüllen auf 25 µl Endvolumen erfolgte durch Zugabe von 2,1 µl Nuklease-freiem Wasser, 5 µl 5x PCR-Puffer, 0,8 µl Taq DNA Polymerase und 2 µl DNA.

Die PCR-Amplifikation wurde mit einem Bio-Rad C1000 Touch Thermal Cycler (Bio-Rad Laboratories, Hercules, USA) durchgeführt. Die PCR-Parameter waren 94°C für 2 min, gefolgt von 32 Zyklen bei 94°C für 30 s, 58°C für 1 min, 68°C für 2 min und einem finalen Elongationsschritt bei 68°C für 10 min. Die amplifizierten PCR-Produkte wurden an einem ABI 3500 Genetic Analyzer (Applied Biosystems, Foster City, USA) analysiert. Hierfür wurden 1,8 µl des PCR-Gemisches, 0,2 µl eines internen Größenstandards (MapMarker Custom, BioVentures Inc., Murfreesboro, USA; Fragmentgrößen: 60, 80, 90, 100, 120, 140, 160, 180, 200, 220, 240, 250, 260, 280, 300, 320, 340, 360, 380, 400, 425, 450, 475, 500, 525 und 550 bp markiert mit dem Fluoreszenzfarbstoff Dy-632) zu 12 µl deionisiertem Formamid zugegeben. Das Gemisch wurde injiziert (15 s), anschließend bei 15 kV und einer Lauftemperatur von 60°C in Performance Optimized Polymer 4 (POP4, Applied Biosystems) aufgetrennt. Die Auswertung der Analysedaten erfolgte mit GeneMapper v5 (Applied Biosystems).

Bei der Erstellung und Strukturierung von Auszuchtpopulationen wird nur eine begrenzte Anzahl an Foundertieren eingesetzt. Daher werden auch nicht alle in der Natur bei Wildmäusen vorkommenden Allele der STR-Marker Eingang in eine Auszuchtkolonie finden. Auszuchtpopulationen besitzen somit im Vergleich zu Wildmäusen eine begrenzte, definierte Anzahl an unterschiedlichen Allelen. Diese Anzahl kann zwischen den verschiedenen Auszuchtpopulationen variieren, ja nach dem welche Foundertiere mit welchen Allelen ursprünglich eingesetzt wurden. Eine Aufgabe ist es, STR-Marker zu identifizieren, die in unterschiedlichen Auszuchtpopulationen möglichst viele verschiedene Allele mit relativ gleichmäßiger Häufigkeitsverteilung aufweisen. Als Referenzwert im Hinblick auf die mögliche Anzahl an Allelen dienen Ergebnisse der Genotypisierung von über 150 unterschiedlichen Tieren aus insgesamt 17 verschiedenen Inzuchtstämmen der Maus.

Zur Bewertung eines autosomalen oder X-chromosomalen Markers werden geeignete Kriterien benötigt, die deren Qualität im Hinblick auf die genetische Vielfalt beschreiben und einen Vergleich zwischen verschiedenen Markern ermöglichen. Dafür geeignete biostatistische Parameter sind der "Polymorphism information content" (PIC) und Heterozygotie (H). Die Heterozygotie errechnet sich aus der Anzahl heterozygoter DNA-Profile (zwei unterschiedliche Allele in der DNA-Probe nachweisbar), dividiert durch die Gesamtanzahl der analysierten DNA-Profile. Bei der Berechnung des Wertes für PIC wird die Anzahl unterschiedlicher Allele sowie deren prozentualer Anteil in der Gesamtpopulation berücksichtigt. Marker, die einen Wert für PIC von 0,50 oder mehr aufweisen, sind für die Beschreibung der genetischen Vielfalt besonders geeignet.

Die hier und in den Ausführungsbeispielen beschriebenen Methoden sind lediglich als Beispiel zu verstehen. Dem Fachmann sind verschiedene weitere Möglichkeiten bekannt, mit deren Hilfe die DNA-Extraktion aus Probenmaterial, das Auffinden von STR-Regionen oder die Generierung und Optimierung spezifischer Oligonukleotid-Primer erreicht werden kann.

**Tabelle 1: Auflistung der erfindungsgemäßen autosomalen und Y-chromosomalen STR-Marker bei der Maus, die eingesetzten Oligonukleotid-Primer, deren chromosomale Lokalisation in Basenpaaren und die Struktur der Wiederholungseinheit des STR-Markers. Die Anzahl an Wiederholungseinheiten bezieht sich auf die in der Datenbank des NCBI hinterlegte Referenzsequenz für den Stamm C57BL/6J. Die Benennung der Allele erfolgte entsprechend den Richtlinien (DNA Recommendations, 1997). Die genomischen DNA-Sequenzen der erfindungsgemäßen STR-Marker sind in Tabelle 2 aufgelistet. (F - Forward-Primer, R - Reverse-Primer)**

| Marker | | Primer Sequenz (5'-3') | Y-chromosomale Position (bp) | Ref. Sequenz, Wiederholungseinheit |
|---|---|---|---|---|
| D1 Mmu121 | | | | |
| SEQ ID NO: 1 | F | | 121856210 - 121856231 | (CTTT)22 (siehe SEQ ID NO: 51) |
| SEQ ID NO: 2 | R | | 121856385-121856363 | |
| D2Mmu008 | | | | |
| SEQ ID NO: 3 | F | | 8370135 - 8370157 | (CTTT)18 (siehe SEQ ID NO: 52) |
| SEQ ID NO: 4 | R | | 8370637 - 8370618 | |
| D3Mmu158 | | | | |
| SEQ ID NO: 5 | F | | 158205109 - 158205130 | (CTTT)22 (siehe SEQ ID NO: 53) |
| SEQ ID NO: 6 | R | | 158205592 - 158205570 | |
| D4Mmu155 | | | | |
| SEQ ID NO: 7 | F | | 155563079 - 155563100 | (GAAA)20 (siehe SEQ ID NO: 54) |
| SEQ ID NO: 8 | R | | 155563258 - 155563238 | |
| D5Mmu108 | | | | |
| SEQ ID NO: 9 | F | | 108460247 - 108460267 | (CTTT)19 (siehe SEQ ID NO: 55) |
| SEQ ID NO: 10 | R | | 108460563 - 108460543 | |
| D6Mmu120 | | | | |
| SEQ ID NO: 11 | F | | 120379001 - 120379025 | (TTTC)15 (siehe SEQ ID NO: 56) |
| SEQ ID NO: 12 | R | | 120379135 - 120379113 | |
| D7Mmu003 | | | | |
| SEQ ID NO: 13 | F | | 3217283 - 3217305 | (GAAA)22 (siehe SEQ ID NO: 57) |
| SEQ ID NO: 14 | R | | 3217426 - 3217404 | |
| D8Mmu127 | | | | |
| SEQ ID NO: 15 | F | | 127974039 - 127974057 | (TTCT)20 TC TTCT (siehe SEQ ID NO: 58) |
| SEQ ID NO: 16 | R | | 127974254 - 127974234 | |
| D9Mmu100 | | | | |
| SEQ ID NO: 17 | F | | 100584836 - 100584858 | (TTTC)3 N (TCTT)19 (siehe SEQ ID NO: 59) |
| SEQ ID NO: 18 | R | | 100585234 - 100585214 | |
| D10Mmu043 | | | | |
| SEQ ID NO: 19 | F | | 43387148 - 43387125 | (GAAA)20 GG (GAAA)3 (siehe SEQ ID NO: 60) |
| SEQ ID NO: 20 | R | | 43386819 - 43386839 | |
| D11 Mmu030 | | | | |
| SEQ ID NO: 21 | F | | 30696089 - 30696110 | (GAAA)19 (siehe SEQ ID NO: 61) |
| SEQ ID NO: 22 | R | | 30696481 - 30696460 | |
| D12Mmu056 | | | | |
| SEQ ID NO: 23 | F | | 56692378 - 56692358 | (CTTT)19 CCTT (CTTT)2 (siehe SEQ ID NO: 62) |
| SEQ ID NO: 24 | R | | 56692087 - 56692108 | |
| D13Mmu096 | | | | |
| SEQ ID NO: 25 | F | | 96579927 - 96579905 | (GAAA)19 (siehe SEQ ID NO: 63) |
| SEQ ID NO: 26 | R | | 96579792 - 96579816 | |
| D14Mmu074 | | | | |
| SEQ ID NO: 27 | F | | 74031224 - 74031244 | (CTTT)17 (siehe SEQ ID NO: 64) |
| SEQ ID NO: 28 | R | | 74031531 - 74031510 | |
| D15Mmu084 | | | | |
| SEQ ID NO: 29 | F | | 84867343 - 84867364 | (GAAA)2GAAG(GAAA)17 (siehe SEQ ID NO: 65) |
| SEQ ID NO: 30 | R | | 84867847 - 84867825 | |
| D16Mmu030 | | | | |
| SEQ ID NO: 31 | F | | 30299393 - 30299372 | (TAAA)7 N (GAAA)16 (siehe SEQ ID NO: 66) |
| SEQ ID NO: 32 | R | | 30299149 - 30299173 | |
| D17Mmu041 | | | | |
| SEQ ID NO: 33 | F | | 41499189 - 41499209 | (AAGA) 17 (siehe SEQ ID NO: 67) |
| SEQ ID NO: 34 | R | | 41499584 - 41499564 | |
| D18Mmu069 | | | | |
| SEQ ID NO: 35 | F | | 69071032 - 69071053 | (CTTT)21 (siehe SEQ ID NO: 68) |
| SEQ ID NO: 36 | R | | 69071314-69071314 | |
| D19Mmu008 | | | | |
| SEQ ID NO: 37 | F | | 8760764 - 8760784 | TTTC TTCC (TTTC)14 (siehe SEQ ID NO: 69) |
| SEQ ID NO: 38 | R | | 8761128 - 8761108 | |
| DYMmu001 | | | | |
| SEQ ID NO: 39 | F | | 334386 - 334406 | (CTTT)21 (siehe SEQ ID NO: 70) |
| SEQ ID NO: 40 | R | | 334525 - 334506 | |
| DYMmu002 | | | | |
| SEQ ID NO: 41 | F | | 2565930 - 2565947 | (CTTT)20 (siehe SEQ ID NO: 71) |
| SEQ ID NO: 42 | R | | 2566201 - 2566182 | |
| DYMmu003 | | | | |
| SEQ ID NO: 43 | F | | 1728782 - 1728801 | (GAAA)8 GAGA (GAAA)3 (siehe SEQ ID NO: 72) |
| SEQ ID NO: 44 | R | | 1729107 - 1729128 | |

**Tabelle 2: Genomische DNA-Sequenzen der erfindungsgemäßen STR-Marker**

| **Marker** | Sequenz |
|---|---|
| **Maus** | |
| D1Mmu121 SEQ ID NO: 51 | |
| D2Mmu008 SEQ ID NO: 52 | |
| D3Mmu 158 SEQ ID NO: 53 | |
| | |
| D4Mmu 155 SEQ ID NO: 54 | |
| D5Mmu 108 SEQ ID NO: 55 | |
| D6Mmu 120 SEQ ID NO: 56 | |
| D7Mmu003 SEQ ID NO: 57 | |
| D8Mmu 127 SEQ ID NO: 58 | |
| D9Mmu 100 SEQ ID NO: 59 | |
| D10Mmu043 | |
| SEQ ID NO: 60 | |
| D11 Mmu030 SEQ ID NO: 61 | |
| D12Mmu056 SEQ ID NO: 62 | |
| D13Mmu096 SEQ ID NO: 63 | |
| D14Mmu074 SEQ ID NO: 64 | |
| D15Mmu084 SEQ ID NO: 65 | |
| | |
| D16Mmu030 SEQ ID NO: 66 | |
| D17Mmu041 SEQ ID NO: 67 | |
| D18Mmu069 SEQ ID NO: 68 | |
| D19Mmu008 SEQ ID NO: 69 | |
| DYMmu001 SEQ ID NO: 70 | |
| DYMmu002 SEQ ID NO: 71 | |
| DYMmu003 SEQ ID NO: 72 | |

| Ratte | |
|---|---|
| DYRno004 SEQ ID NO: 73 | |
| DYRno004a SEQ ID NO: 74 | |
| DYRno165 SEQ ID NO: 75 | |
| DYRno304 | |
| SEQ ID NO: 76 | |

Die Erfindung wird nachfolgend anhand von 1 Zeichnung und 6 Ausführungsbeispielen näher erläutert.

Es zeigt:

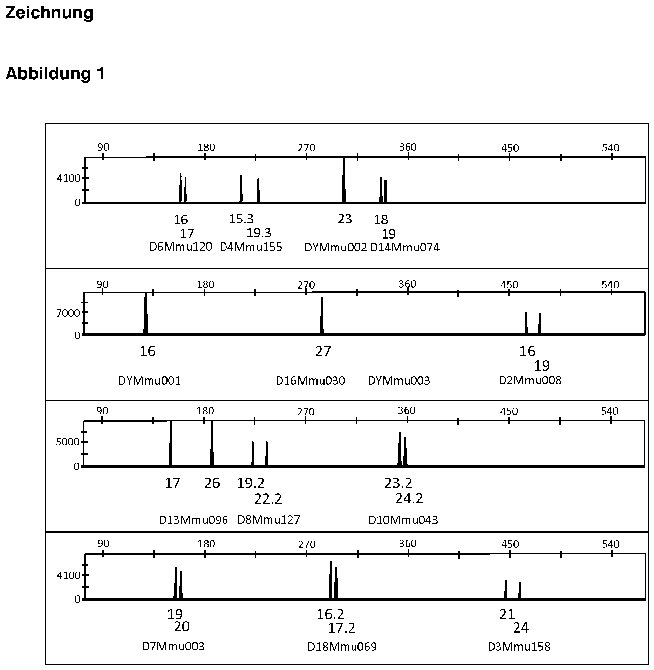

- **Figur 1**: ein Elektropherogramm, welches die Ko-Amplifikation von 11 autosomalen STR-Markern und der Y-chromosomalen Marker DYMmu001, DYMmu002 und DYMmu003 einer DNA-Probe vom Typ M. m. domesticus darstellt. Die PCR-Produkte der STR-Marker sind mit folgenden Fluoreszenzfarbstoffen markiert und innerhalb eines Farbstoffes der Größe nach aufsteigend sortiert: Farbstoff 6FAM^{™}: D6Mmu120, D4Mmu155, DYMmu002, D14Mmu074; Farbstoff DY530^{™}: DYMmu001, D16Mmu030, DYMmu003, D2Mmu008; Farbstoff ATTO550^{™}: D13Mmu096, D8Mmu127, D10Mmu043; Farbstoff DY510XL^{™}: D7Mmu003, D18Mmu069, D3Mmu158. Der zur Berechnung der Fragmentlängen eingesetzte Größenstandard ist mit dem Farbstoff DY632^{™} markiert.

### Ausführungsbeispiel 1: Zuordnung der Y-Chromosomen bei ausgewählten Auszuchtpopulationen zu den Typen M. m. musculus und M. m. domesticus.

Die Beschreibung der genetischen Vielfalt einer Auszuchtpopulation bedarf der Kenntnis über die Anzahl an unterschiedlichen Varianten des Y-Chromosoms in der Population. Diese Aufgabe wird dadurch gelöst, indem zunächst eine Zuordnung der Y-Chromosomen von Auszuchtpopulationen zu den beiden Typen M. m. domesticus und M. m. musculus vorgenommen wird. Hierfür werden Marker benötigt, die ein Y-Chromosom zu einer der beiden Grundvarianten zuordnen können. Überraschend konnte der STR-Marker DYMmu003 identifiziert werden, welcher bei verschiedenen Varianten von M. m. musculus unterschiedliche Allele besitzt. Die eingesetzten PCR-Primer weisen dagegen bei allen Varianten des Y-Chromosoms vom Typ M. m. domesticus keine homologe Sequenz auf. Dies führt dazu, dass im Falle des Vorliegens eines Chromosoms vom Typ M. m. domesticus kein PCR-Produkt generiert wird. DYMmu003 wird zur Feststellung der Zugehörigkeit von Y-Chromosomen zu einem der beiden Typen genutzt. Hierfür wurden jeweils zwischen 5 und 25 männliche Individuen der Auszuchtpopulationen Hsd:ICR(CD1), Crl:CD1(ICR), RjOrl:SWISS, Crl:NMRI, Han:NMRI, RjHan:NMRI und HsdWin:NMRI analysiert. Insgesamt 100 männliche Tiere von 7 verschiedenen Auszuchtpopulationen der Maus wurden mit dem STR-Marker DYMmu003 analysiert und entsprechend zu M. m. musculus oder M. m. domesticus zugeordnet (**Tabelle 3**). Bei Typ M. m. domesticus werden für keinen der beiden Marker DNA-Produkte erhalten, bei Typ M. m. musculus werden stets Allele detektiert. Völlig unerwartet besitzt lediglich die Auszuchtpopulation Crl:NMRI Y-Chromosomen beider Typen, wogegen die Auszuchtpopulationen Hsd:ICR(CD1), Crl:CD1(ICR), RjOrl:SWISS sowie RjHan:NMRI ausschließlich Varianten vom Typ M. m. domesticus aufweisen und Han:NMRI sowie HsdWin:NMRI stets nur dem Typ M. m. musculus zugeordnet werden können.

**Tabelle 3: Zuordnung von Y-Chromosomen zum Typ M.m. domesticus und M. m. musculus bei verschiedenen Auszuchtpopulationen (N = Anzahl der getesteten Individuen)**

| | | M.m. domesticus | M. m. musculus |
|---|---|---|---|
| | N | | |
| Hsd:ICR(CD1) | 25 | 25 | 0 |
| Crl:CD1(ICR) | 5 | 5 | 0 |
| RjOrl:SWISS | 20 | 20 | 0 |
| Crl:NMRI | 20 | 17 | 3 |
| Han:NMRI | 20 | 0 | 20 |
| RjHan:NMRI | 5 | 5 | 0 |
| HsdWin:NMRI | 5 | 0 | 5 |

### Ausführungsbeispiel 2: Zuordnung von Allelen der STR-Marker DYMmu001, DYMmu002 und DYMmu003 des Y-Chromosoms bei ausgewählten Auszuchtpopulationen

Aufgabe der vorliegenden Erfindung ist weiterhin, STR-Marker für das Y-Chromosom zur Verfügung zu stellen, die sowohl beim Typ M. m. musculus als auch beim Typ M. m. domesticus polymorph sind und für die Beschreibung von Y-chromosomalen Haplotypen eingesetzt werden können. Die erfindungsgemäßen STR-Marker DYMmu001 und DYMmu002 besitzen diese Eigenschaft. Anhand von DNA-Proben männlicher Tiere verschiedener Inzuchtstämme und Auszuchtpopulationen der Maus wurde das mögliche Allelspektrum für die Marker DYMmu001, DYMmu002 und DYM003 bestimmt

Insgesamt 50 männliche Tiere von 15 verschiedenen Inzuchtstämmen und 85 männliche Tiere von 7 verschiedenen Auszuchtpopulationen der Maus wurden mit den Markern DYMmu001, DYMmu002 und DYMmu003 analysiert **(Tabelle 4**). Die Zuordnung zu M. m. musculus oder M. m. domesticus bei den Auszuchtpopulationen erfolgte anhand der entsprechenden Untersuchungsergebnisse für DYMmu003. Einige Allele wurden ausschließlich bei Inzuchtstämmen nachgewiesen.

**Tabelle 4: Nachgewiesene Allele der STR-Marker DYMmu001, DYMmu002 und DYMmu003 sowie deren Zuordnung bei Auszuchtpopulationen zu M. m. musculus und M. m. domesticus**

| | Inzucht + Auszucht | Auszucht | |
|---|---|---|---|
| | Gefundene Allele | M.m. domesticus | M. m. musculus |
| DYMmu001 | 16, 17, 18, 19, 21, 20, 22, 23, 24, 25, 26 | 16, 17, 18, 20, 21 | 21, 22, 23 |
| DYMmu002 | 17, 19, 20, 21, 23, 24, 25 | 17, 23, 24, 25 | 19, 20 |
| DYMmu003 | 12, 14 | kein PCR-Produkt | 14 |

### Ausführungsbeispiel 3: Nachweis unterschiedlicher Y-chromosomaler Haplotypen innerhalb von Auszuchtpopulationen der Maus

Da das Y-Chromosom nicht rekombiniert, können die Analyseergebnisse unterschiedlicher Y-STR-Marker miteinander kombiniert und in Form von Haplotypen dargestellt werden. In **Tabelle** 5 werden unterschiedliche **Y-chromosomale Haplotypen** für 7 verschiedene Auszuchtpopulationen von CD1, SWISS und NMRI dargestellt, die anhand der Analyseergebnisse für die STR-Marker DYMmu001, DYMmu002 und DYMmu003 erhalten wurden. Mit Ausnahme der Auszuchtpopulation HsdWin:NMRI mit 5 identischen Y-STR-Haplotypen unter 5 getesteten Individuen besitzen alle anderen Auszuchtpopulationen mehr als einen Haplotyp. Innerhalb einer konkreten Auszuchtpopulation eine Zuordnung von männlichen Individuen zu unterschiedlichen Haplotypen vorgenommen werden, ebenso die Zuordnung zu einem der beiden Grundtypen des Y-Chromosoms M. m. musculus oder M. m. domesticus. Dieser Ansatz zur Beschreibung der genetischen Vielfalt von Auszuchtpopulationen über das Y-Chromosom ist neu und kann zukünftig zum genetischen Monitoring eingesetzt werden.

Insgesamt 100 männliche Tiere von 7 verschiedenen Auszuchtpopulationen der Maus wurden mit den STR-Markern DYMmu001, DYMmu002 und DYMmu003 analysiert. Aus den Ergebnissen der Analyse der Einzelproben wurden die Haplotypen abgeleitet, wobei die Ergebnisse im Haplotyp in der Reihenfolge DYMmu001-DYMmu002-DYMmu003 darstellt sind. Der Wert "0" für DYMmu003 zeigt an, dass kein PCR-Produkt nachweisbar ist und dass es sich um ein Chromosom vom Typ M. m. domesticus handelt. Innerhalb einer Auszuchtpopulation können unterschiedliche Kombinationen von Allelen nachgewiesen werden, was die Anwesenheit verschiedener, voneinander unterscheidbarer Y-Chromosomen innerhalb einer Auszuchtpopulation belegt.

Der Abgleich von Haplotypen zwischen verschiedenen Auszuchtpopulationen zeigt, dass zum Teil Haplotypen vorliegen, die einmalig für eine konkrete Auszuchtpopulation sind und in keiner anderen Auszuchtpopulation nachgewiesen werden konnten.

**Tabelle 5: Y-chromosomale Haplotypen bei verschiedenen Auszuchtpopulationen. Die Reihenfolge der Angabe im Haplotyp ist DYMmu001 - DYMmu002 - DYMmu003**

| Hsd:ICR (CD1) N=25 | Crl:CD1 (ICR) N=5 | RjOrl:SW ISS N=20 | Crl:NMRI N=20 | Han:NMRI N=20 | RjHan:NM RI N=5 | HsdWin: NMRI N=5 |
|---|---|---|---|---|---|---|
| 16-23-0 | 17-24-0 | 16-23-0 | 19-17-0 | 21-19-14 | 17-24-0 | 23-20-14 |
| 16-24-0 | 17-25-0 | 17-23-0 | 20-17-0 | 21-20-14 | 17-25-0 | |
| 17-23-0 | 18-24-0 | 17-24-0 | 21-17-0 | | | |
| 17-24-0 | | | 22-20-14 | | | |
| 17-25-0 | | | | | | |

### Ausführungsbeispiel 4: Charakterisierung des Y-Chromosoms der Ratte mit Hilfe von STR-Markern

Männliche Tiere von 17 verschiedenen Stämmen von Rattus norwegicus wurden mit den Y-chromosomalen Markern DYRno004, DYRno165 und DYRno304 analysiert. Die Bezeichnung der Allele wurde anhand der gemessenen Längen der Amplifikationsprodukte vorgenommen. Dies ist ausreichend, um die Vielfalt an unterschiedlichen Allelen zu dokumentieren. Die exakte Kenntnis der DNA-Sequenzen der einzelnen Allele ist hierfür nicht zwingend erforderlich. Die aus den Analyseergebnissen abgeleiteten Haplotypen sind in der **Tabelle** 7 in der Reihenfolge DYRno004, DYRno165 und DYRno304 dargestellt. Alle Stämme sind voneinander unterscheidbar. Somit eignen sich diese Marker für die Genotypisierung des Y-Chromosoms der Ratte sowie zur Feststellung der genetischen Vielfalt innerhalb von Auszuchtpopulationen der Ratte. In **Tabelle** 6 sind die erfindungsgemäßen STR-Marker, die eingesetzten Oligonukleotid-Primer, deren chromosomale Lokalisation und die Struktur der Wiederholungseinheiten zusammengefasst. Für jeden Primer wird dessen chromosomale Lokalisation in Basenpaaren angegeben, die auf der Basis der Referenz-DNA (NCBI, Version Rnor_6.0) ermittelt wurde. Da das Y-Chromosom der Ratte nur sehr kurz ist, wurde zur Kennzeichnung der chromosomalen Position des Markers eine detailliertere Zuordnung vorgenommen. DYRno165 entspricht der Position bei 1,65 Megabasen.

Das Oligonukleotid-Primerpaar des Markers DYRno004 bindet spezifisch an zwei verschiedene Targetregionen des Y-Chromosoms und für jede Targetregion wird ein Allelprodukt erhalten. Die dabei amplifizierten beiden Allele unterscheiden sich meist voneinander hinsichtlich der Anzahl an Wiederholungseinheiten, so dass mit einem Primerpaar gleichzeitig zwei verschiedene Allele detektiert werden können. Die Zuordnung der einzelnen Allele zur konkreten Targetregion ist dabei nicht möglich. Der STR-Marker DYRno004 stellt somit einen Doppelmarker dar, der sehr polymorph ist und sich für die Erstellung eines Y-spezifischen Haplotyps ideal eignet. Das kleinere der beiden Allele wird zuerst im Haplotyp aufgeführt, anschließend das zweite, größere Allel. Für die beiden Marker DYRno165 und DYRno304 wird ein und derselbe reverse Primer eingesetzt, Dank der unterschiedlichen Forward-Primer kann eine eindeutige Zuordnung von Allelen zum konkreten Marker vorgenommen werden. Erfindungsgemäß können die beiden spezifischen Forward-Primer mit verschiedenen Fluoreszenzfarbstoffen versehen werden, was eine eindeutige Zuordnung der Amplifikationsprodukte ermöglicht.

**Tabelle 6: Auflistung der erfindungsgemäßen Y-chromosomalen STR-Marker bei der Ratte Rattus norwegicus, die eingesetzten Oligonukleotid-Primer, deren Lokalisation auf dem Y-Chromosom in Basenpaaren und das Muster der STR-Wiederholungseinheit. Die Anzahl an Wiederholungseinheiten bezieht sich auf die in der Datenbank des NCBI (Version Rnor_6.0) hinterlegte Referenzsequenz. Die Benennung der Allele erfolgte entsprechend den Richtlinien (DNA Recommendations, 1997). (F - Forward-Primer, R - Reverse-Primer)**

| Marker | | Primer Sequenz (5'-3') | Y-chromosomale Position (bp) | Wiederholungseinheit |
|---|---|---|---|---|
| DYRno004 | | | | |
| SEQ ID NO: 45 | F | | 49153 -49172 249767 - 249748 | CTTT (CCTT)3 (CTTT) 18 (siehe SEQ ID NO: 73 und 74) |
| SEQ ID NO: 46 | R | | 49393 - 49374 249523 - 249542 | |
| DYRno165 | | | | |
| SEQ ID NO: 47 | F | | 1657537 - 1657557 | (ATCTATGT)7 ATCT (TCTA)15 (siehe SEQ ID NO: 75) |
| SEQ ID NO: 48 | R | | 1657766 - 1657746 | |
| DYRno304 | | | | |
| SEQ ID NO: 49 | F | | 3047184 - 3047203 | (ATCT)16 (siehe SEQ ID NO: 76) |
| SEQ ID NO: 50 | R | | 3047359 - 3047339 | |

In **Tabelle 7** sind die Analyseergebnisse der Genotypisierung von 17 verschiedenen Stämmen von Rattus norwegicus mit den STR-Markern DYRno004, DYRno165 und DYRno304 zusammengefasst. Die Bezeichnung der Allele entspricht den gemessenen Längen der Amplifikationsprodukte. Dies ist prinzipiell ausreichend, um ohne konkrete Kenntnis der zugrunde liegenden DNA-Sequenz die Vielfalt an unterschiedlichen Allelen zu belegen. Die dargestellten Haplotypen sind in der Reihenfolge der Analyseergebnisse für DYRno004, DYRno165 und DYRno304 dargestellt. Jeder Stamm weist seinen eigenen Haplotypen auf. Die Kombination der drei erfindungsgemäßen STR-Marker ist geeignet, die genetische Vielfalt des Y-Chromosoms bei der Ratte abzubilden.

**Tabelle 7: Y-chromosomale Haplotypen bei verschiedenen Stämmen der Ratte**

| Rattenstamm | STR Loci | | | Haplotyp |
|---|---|---|---|---|
| | DYRno004 | DYRno165 | DYRno304 | |
| ACI | 255-258 | 243 | 188 | 255 - 258 - 243 - 188 |
| BN | 254-264 | 231 | 184 | 254-264-231 - 184 |
| BS | 259-264 | 243 | 192 | 259 - 264 - 243 -192 |
| DA | 234-247 | 247 | 192 | 234 - 247 - 247 -192 |
| E3 | 259-267 | 247 | 192 | 259 - 267 - 247 -196 |
| F344 | 255-267 | 243 | 196 | 255 - 267 - 243 -196 |
| LE | 259-267 | 247 | 196 | 259 - 267 - 247 -196 |
| LEW | 259-264 | 251 | 188 | 259 - 264 - 251 - 188 |
| LOU-C | 264-267 | 247 | 192 | 264 - 267 - 247 - 192 |
| MNS | 255-271 | 247 | 192 | 255 - 271 - 247 - 192 |
| NAR | 255-259 | 247 | 192 | 255 - 259 - 247 - 192 |
| OM | 251-254 | 231 | 184 | 251 - 267 - 231 - 184 |
| PAR | 251-267 | 243 | 196 | 251 - 267 - 243 - 196 |
| PVG | 251-264 | 251 | 211 | 251 - 264 - 251 - 211 |
| WC | 264-275 | 239 | 196 | 264 - 275 - 239 - 196 |
| WF | 255-255 | 247 | 192 | 255 - 255 - 247 - 192 |
| WKY | 259-283 | 255 | 188 | 259 - 283 - 255 - 188 |

### Ausführungsbeispiel 5: Bestimmung des Genotyps für Tiere der Auszuchtpopulationen Hsd:ICR(CD1) und RjOrl:SWISS

Es wurden für jeweils 20 männliche Tiere der Auszuchtpopulationen **Hsd:ICR(CD1)** und RjOrl:SWISS die Genotypen bestimmt und darauf basierend die Werte für PIC und Het berechnet. Wesentlich für die Bewertung der Qualität eines Markers ist, dass mehrere unterschiedliche Allele nachweisbar sind und keines der Allele mehr als 50% der Allele in einer Population repräsentieren sollte. Um das einschätzen zu können, wurde die Analyse von 20 Tieren pro Population als ausreichend erachtet. Bei einer solchen Anzahl an Tieren liegen Ergebnisse über insgesamt 40 Allele vor (2 pro Tier), ein Allel mit einem Anteil von 10% in der Population wird dabei statistisch gesehen viermal nachgewiesen werden.

Die nach der Genotypisierung einer Vielzahl an Inzuchtstämmen und Auszuchtpopulationen ermittelte Anzahl an möglichen Referenzallelen variiert je nach Marker zwischen 7 und 11. Für Hsd:ICR(CD1) und RjOrl:SWISS wurden STR-Marker identifiziert, bei denen unter den 20 genotypisierten Tieren das Vorliegen von mindestens 6 bis 9 verschiedenen Allelen nachgewiesen werden konnte. Die Werte für PIC variieren zwischen 0,34 und 0,84. Die Heterozygotie-Rate liegt zwischen 0,50 und 0,95.

**Tabelle 8: Charakterisierung von STR-Markern bei RjOrl:SWISS und Hsd:ICR(CD1) anhand der Anzahl an nachgewiesenen Allelen pro Marker (No.) und der biostatistischen Parameter "Polymorphism information content" (PIC) sowie Heterozygotie (Het). In der Spalte "Refer. Allele" ist die Gesamtzahl an Allelen dargestellt, die bei 150 Individuen verschiedener Inzuchtund Auszuchtstämme sowie Substämmen von Inzuchtstämmen der Maus insgesamt beobachtet wurden. In der Spalte "No." ist angegeben, wie viele unterschiedliche Allele unter den 20 DNA-Proben von männlichen Individuen der konkreten Auszuchtpopulation nachgewiesen wurden.**

| STR Locus | Refer. Allele | | RjOrl:SWISS | | | | Hsd:ICR(CD1) | | |
|---|---|---|---|---|---|---|---|---|---|
| | | | No. | PIC | Het | | No. | PIC | Het |
| D1Mmu121 | 10 | | 8 | 0,64 | 0,75 | | 6 | 0,70 | 0,85 |
| D2Mmu008 | 7 | | 6 | 0,67 | 0,65 | | 6 | 0,75 | 0,75 |
| D3Mmu158 | 8 | | 7 | 0,79 | 0,90 | | 6 | 0,69 | 0,60 |
| D4Mmu155 | 8 | | 6 | 0,72 | 80,0 | | 6 | 0,69 | 0,75 |
| D5Mmu108 | 8 | | 6 | 0,63 | 70,0 | | 6 | 0,71 | 0,95 |
| D6Mmu120 | 7 | | 7 | 0,75 | 0,85 | | 7 | 0,79 | 0,80 |
| D7Mmu003 | 9 | | 6 | 0,72 | 0,70 | | 7 | 0,75 | 0,85 |
| D8Mmu127 | 10 | | 7 | 0,74 | 0,75 | | 7 | 0,63 | 0,65 |
| D9Mmu100 | 10 | | 5 | 0,50 | 0,45 | | 8 | 0,83 | 0,90 |
| D10Mmu043 | 7 | | 7 | 0,80 | 0,75 | | 6 | 0,73 | 0,70 |
| D11Mmu030 | 11 | | 6 | 0,71 | 0,85 | | 4 | 0,57 | 0,75 |
| D12Mmu056 | 8 | | 7 | 0,78 | 0,80 | | 4 | 0,60 | 0,70 |
| | | | | | | | | | |
| D13Mmu096 | 11 | | 9 | 0,84 | 0,90 | | 7 | 0,79 | 0,85 |
| D14Mmu074 | 8 | | 6 | 0,72 | 0,85 | | 4 | 0,64 | 0,75 |
| D15Mmu084 | 9 | | 9 | 0,73 | 0,65 | | 5 | 0,52 | 0,50 |
| D16Mmu030 | 10 | | 6 | 0,72 | 0,85 | | 5 | 0,71 | 0,75 |
| D17Mmu041 | 7 | | 5 | 0,61 | 0,75 | | 3 | 0,34 | 0,55 |
| D18Mmu069 | 11 | | 7 | 0,78 | 0,65 | | 7 | 0,67 | 0,70 |
| D19Mmu008 | 8 | | 7 | 0,72 | 0,70 | | 5 | 0,51 | 0,65 |

### Ausführungsbeispiel 6: Elektropherogramm einer Multiplex-PCR von 14 STR-Loci

In Figur 1 ist ein Elektropherogramm einer Multiplex-PCR von 14 STR-Loci dargestellt, bei welcher die DNA-Probe eines männlichen Tieres von **Hsd:ICR(CD1)** analysiert wurde. Jeweils ein Primer eines Oligonukleotid-Pärchens wurde mit folgendem Farbstoff kovalent gekoppelt: Farbstoff 6FAM^{™}: D6Mmu120, D4Mmu155, DYMmu002, D14Mmu074; Farbstoff DY530^{™}: DYMmu001, D16Mmu030, DYMmu003, D2Mmu008; Farbstoff ATTO550^{™}: D13Mmu096, D8Mmu127, D10Mmu043; Farbstoff 'DY510XL^{™}: D7Mmu003, D18Mmu069, D3Mmu158. Der zur Berechnung der Fragmentlängen eingesetzte Größenstandard ist mit dem Farbstoff DY632^{™} markiert.

Die PCR wurde mittels Snooplex^{®} FastPrep PCR Reagenzien (GVG Genetic Monitoring, Leipzig) in einem Gesamtvolumen von 25 µl durchgeführt. Der Reaktionsansatz für die PCR-Analyse bestand aus folgenden Komponenten: 2,1 µl Nuklease-freies Wasser, 5 µl 5x PCR-Puffer, 0,8 µl Taq DNA Polymerase, Primergemische der STR-Marker D2Mmu008 (0,8 µl), D3Mmu158 (1,2 µl), D4Mmu155 (0,8µl), D6Mmu120 (1,6 µl), D7Mmu003 (1,2 µl), D8Mmu127 (1,2 µl), D10Mmu043 (0,4 µl), D13Mmu096 (0,7 µl), D14Mmu074 (1,6 µl), D16Mmu030 (1,6 µl), D18Mmu069 (1,2 µl), DYMmu001 (1,0 µl), DYMmu002 (0,8 µl), DYMmu003 (1,0 µl), 2 µl DNA.

Die in Ausführungsbeispiel 6 gezeigte erfindungsgemäße Multiplex-PCR führt zu keinen nachweisbaren Allelen beim Einsatz von DNA humanen Ursprungs, der Ratte, Hamster oder Zebrafisch.

### Referenzen

Rapp KG (1972) HAN-rotation, a new system for rigorous outbreeding. Z. Versuchstierkunde 14: 133-142

Pertile MD, Graham AN, Choo KH, Kalitsis P (2009) Rapid evolutionof mouse Y centromere repeat DNA belies recent sequence stability. Genome Res 19(12):2202-2213

Nelson VR, Spezio SH, Nadeau JH (2010) Transgenerational genetic effects of the paternal Y chromosome on daughters' phenotypes. Epigenomics 2 (4): 513-521

Kluge R, Wedekind D (2016) Auszuchtstämme. Zuchtmethoden und genetische Eigenschaften. Fachinformation aus dem Ausschuss für Genetik und Labortierzucht. GV-SOLAS

Yalcin B, Nicod J, Bhomra A, Davidson S, Cleak J, Farinelli L, Osteras M, Whitley A, Yuan W, Gan X (2010) Commercially available outbred mice for genome-wide association studies. PLoS Genet 6(9). pii: e1001085

Witmer PD, Doheny KF, Adams MK, Boehm CD, Dizon JS, Goldstein JL, Templeton TM, Wheaton AM, Dong PN, Pugh EW, Nussbaum RL, Hunter K, Kelmenson JA, Rowe LB, Brownstein MJ (2003) The development of a highly informative mouse simple sequence length polymorphism (SSLP) marker set and construction of a mouse family tree using parsimony analysis. Genome Research 13:485-491

US 2014/0066322

WO 2016/008894

Shang HT, Wei H, Yue BF, Xu P (2008) Microsatellite analysis in Wistar and Spague-Darley ourbred rats. Fen Zi Xi Bao Sheng Wu Xue Bao. 41(1):28-34. In Chinese, Abstract in English Clapcote SJ and Roder JC (2006) Deletion polymorphism of Disc1 is common to all 129 Mouse substrains: Implications for gene targeting studies of brain function. Genetics 173: 2407-2410.

Kalendar R, Lee D, Schulman AH 2014. FastPCR software for PCR, in silico PCR, and oligonucleotide assembly and analysis. DNA Cloning and Assembly Methods, Methods in Molecular Biology, Svein Valla and Rahmi Lale (ed.), Humana Press, 1116: 271-302 (ISBN 978-1-62703-763-1).

DNA recommendations - further report of the DNA Commission of the ISFH regarding the use of short tandem repeat systems (1997) Forensic Sci Int 87: 179-184

## Patentansprüche

1. Methode zur genetischen Identifizierung und/oder zur Unterscheidung von zwei oder mehr Tieren der Spezies Maus, Ratte, Hamster und Zebrafisch aus unterschiedlichen Auszuchtstämmen, des identischen Auszuchtstammes oder von Substämmen des identischen Auszuchtstammes, umfassend die folgenden Teilschritte:
(a) Verwendung von mindestens einer DNA-Probe, die analysiert werden soll;
(b) Auswahl eines Sets von Loci für die DNA-Probe, welche mindestens 6, 7 oder 8, besonders bevorzugt mindestens 9 oder 10, am meisten bevorzugt mindestens 11 autosomale STR-Marker umfasst, die gleichzeitig in einem Reaktionsansatz ko-amplifiziert werden, wobei die autosomalen STR-Loci durch einen hohen Wert für den "Polymorphism information content" charakterisiert werden, der im Bereich von 0,50 bis 0,99, besonders bevorzugt im Bereich zwischen 0,60 bis 0,99 liegt;
(c) Auswahl eines Sets von Loci für die DNA-Probe, welche mindestens zwei Y-chromosomale STR-Marker umfasst, die gleichzeitig mit den autosomalen STR-Loci (b) in einem Reaktionsansatz ko-amplifiziert werden;
(d) Verwendung eines Sets von Oligonukleotid-Primerpaaren, bei denen jedes Primerpaar spezifisch an die flankierenden DNA-Abschnitte eines STR-Markers aus den Sets der STR-Loci (b) und (c) bindet;
(e) Ko-Amplifikation der für das Set ausgewählten Loci, wobei das Reaktionsprodukt ein Gemisch darstellt, bestehend aus amplifizierten Allelen von jedem der koamplifizierten STR-Loci der Sets (b) und (c);
(f) Analyse des Allelgemisches und Bestimmung und Zuordnung der spezifischen Allele für jeden der amplifizierten Loci der Sets (b) und (c) für die gegebene DNA-Probe.

2. Methode nach Anspruch 1, **gekennzeichnet dadurch dass** mindestens zwei der mindestens 6 autosomalen STR-Loci (b) ausgewählt sind aus der Gruppe umfassend die Loci D1Mmu121 (SEQ ID NO: 51), D2Mmu008 (SEQ ID NO: 52), D3Mmu158 (SEQ ID NO: 53), D4Mmu155 (SEQ ID NO: 54), D5Mmu108 (SEQ ID NO: 55), D6Mmu120 (SEQ ID NO: 56), D7Mmu003 (SEQ ID NO: 57), D8Mmu127 (SEQ ID NO: 58), D9Mmu100 (SEQ ID NO: 59), D10Mmu043 (SEQ ID NO: 60), D11Mmu030 (SEQ ID NO: 61), D12Mmu056 (SEQ ID NO: 62), D13Mmu096 (SEQ ID NO: 63), D14Mmu074 (SEQ ID NO: 64), D15Mmu084 (SEQ ID NO: 65), D16Mmu030 (SEQ ID NO: 66), D17Mmu041 (SEQ ID NO: 67), D18Mmu069 (SEQ ID NO: 68) und D19Mmu008 (SEQ ID NO: 69).

3. Methode nach Anspruch 1 und 2, **gekennzeichnet dadurch dass** das Set aus mindestens elf autosomalen Loci in Schritt (b) die Loci D2Mmu008 (SEQ ID NO: 52), D3Mmu158 (SEQ ID NO: 53), D4Mmu155 (SEQ ID NO: 54), D6Mmu120 (SEQ ID NO: 56), D7Mmu003 (SEQ ID NO: 57), D8Mmu127 (SEQ ID NO: 58), D10Mmu043 (SEQ ID NO: 60), D13Mmu096 (SEQ ID NO: 63), D14Mmu074 (SEQ ID NO: 64), D16Mmu030 (SEQ ID NO: 66) und D18Mmu069 (SEQ ID NO: 68) umfasst.

4. Methode nach Anspruch 1, weiterhin umfassend die Identifizierung des Geschlechts in der DNA-Probe des Individuums in Schritt (a) anhand des Sets von Loci gemäß Schritt (c).

5. Methode nach Anspruch 4, weiterhin umfassend die Unterscheidung von Y-Chromosomen vom Typ Mus musculus musculus und vom Typ Mus musculus domesticus anhand des Sets von Loci gemäß Schritt (c).

6. Methode nach Anspruch 4, **gekennzeichnet dadurch, dass** der Locus zur Bestimmung des Geschlechts einen polymorphen STR-Locus der Maus darstellt, der Y-chromosomale Haplotypen unterscheiden kann und aus einer Gruppe von nachfolgend aufgelisteten Loci ausgewählt wird: DYMmu001 (SEQ ID NO: 70), DYMmu002 (SEQ ID NO: 71) und DYMmu003 (SEQ ID NO: 72).

7. Methode nach Anspruch 4, **gekennzeichnet dadurch dass** der Locus zur Bestimmung des Geschlechts einen polymorphen STR-Locus der Ratte darstellt, der Y-chromosomale Haplotypen unterscheiden kann und aus einer Gruppe von nachfolgend aufgelisteten Loci ausgewählt wird: DYRno004 (SEQ ID NO: 73 und SEQ ID NO: 74), DYRno165 (SEQ ID NO: 75) und DYRno304 (SEQ ID NO: 76).

8. Methode nach Anspruch 1, **gekennzeichnet dadurch dass** eine Multiplex-Amplifikation mit mindestens dreizehn Paaren an Oligonukleotid-Primern durchgeführt wird, welche mindestens elf autosomale und zwei Y-chromosomale STR-Loci umfasst.

9. Methode nach Anspruch 1, **gekennzeichnet dadurch dass** mindestens ein Oligonukleotid-Primer eines Primerpaares kovalent mit einem Detektionsfarbstoff gekoppelt wird, vorzugsweise mit einem Fluoreszenzfarbstoff.

10. Methode nach Anspruch 9, **gekennzeichnet dadurch dass** mindestens vier verschiedene Farbstoff-gekoppelte Primer mit vier verschiedenen Detektionsfarbstoffen, vorzugsweise vier verschiedenen Fluoreszenzfarbstoffen eingesetzt werden.

11. Methode nach Anspruch 1, **gekennzeichnet dadurch dass** die Zuordnung der amplifizierten Allele in Schritt (f) anhand des Vergleiches mit einem Größenstandard erfolgt, wobei der Größenstandard ein Gemisch aus DNA-Fragmenten mit bekannter Größe und/oder ein Locus-spezifisches Gemisch bekannter Allele darstellt.

12. Kit zur genetischen Identifizierung und/oder zur Unterscheidung von zwei oder mehr Tieren aus DNA-Extrakten von Individuen aus Wildtyp-Populationen, aus unterschiedlichen Inzucht- oder Auszuchtstämmen, des identischen Inzucht- oder Auszuchtstammes oder von Substämmen des identischen Inzucht- oder Auszuchtstammes der Maus, umfassend:
(a) ein Set von Oligonukleotid-Primerpaaren, bei denen jedes Primerpaar spezifisch an die flankierenden DNA-Abschnitte eines Locus aus dem Set der STR-Loci bindet und welches mindestens 6, 7 oder 8, besonders bevorzugt mindestens 9 oder 10, am meisten bevorzugt mindestens 11 autosomale STR-Marker umfasst, die gleichzeitig in einem Reaktionsansatz ko-amplifiziert werden können, wobei die autosomalen STR-Loci durch einen hohen Wert für den "Polymorphism information content" charakterisiert werden, der im Bereich von 0,50 bis 0,99, besonders bevorzugt im Bereich zwischen 0,60 bis 0,99 liegt;
(b) ein Set von Oligonukleotid-Primerpaaren, die spezifisch an die flankierenden DNA-Abschnitte des Y-Chromosoms binden und welches mindesten zwei, vorzugsweise drei Y-chromosomale STR-Marker umfasst, die gleichzeitig mit den autosomalen STR-Loci (a) in einem Reaktionsansatz ko-amplifiziert werden können;
(c) Reagenzien, die für die Durchführung von mindestens einer Multiplex-PCR ausreichend sind; und
(d) einen Größenstandard, welcher unterschiedliche DNA-Marker mit einer bekannten Fragmentlänge enthält.

13. Kit nach Anspruch 12, **gekennzeichnet dadurch dass** mindestens zwei der mindestens 6 autosomalen STR-Loci (a) ausgewählt sind aus der Gruppe umfassend die Loci D1Mmu121 (SEQ ID NO: 51), D2Mmu008 (SEQ ID NO: 52), D3Mmu158 (SEQ ID NO: 53), D4Mmu155 (SEQ ID NO: 54), D5Mmu108 (SEQ ID NO: 55), D6Mmu120 (SEQ ID NO: 56), D7Mmu003 (SEQ ID NO: 57), D8Mmu127 (SEQ ID NO: 58), D9Mmu100 (SEQ ID NO: 59), D10Mmu043 (SEQ ID NO: 60), D11Mmu030 (SEQ ID NO: 61), D12Mmu056 (SEQ ID NO: 62), D13Mmu096 (SEQ ID NO: 63), D14Mmu074 (SEQ ID NO: 64), D15Mmu084 (SEQ ID NO: 65), D16Mmu030 (SEQ ID NO: 66), D17Mmu041 (SEQ ID NO: 67), D18Mmu069 (SEQ ID NO: 68) und D19Mmu008 (SEQ ID NO: 69).

14. Kit nach Anspruch 12, **gekennzeichnet dadurch dass** das Set aus mindestens elf autosomalen Loci in Schritt (a) die Loci D2Mmu008 (SEQ ID NO: 52), D3Mmu158 (SEQ ID NO: 53), D4Mmu155 (SEQ ID NO: 54), D6Mmu120 (SEQ ID NO: 56), D7Mmu003 (SEQ ID NO: 57), D8Mmu127 (SEQ ID NO: 58), D10Mmu043 (SEQ ID NO: 60), D13Mmu096 (SEQ ID NO: 63), D14Mmu074 (SEQ ID NO: 64), D16Mmu030 (SEQ ID NO: 66) und D18Mmu069 (SEQ ID NO: 68) umfasst.

15. Kit nach einem der Ansprüche 12 bis 14, **gekennzeichnet dadurch, dass** das Set aus Y-chromosomalen Loci (b) die Loci DYMmu001 (SEQ ID NO: 70), DYMmu002 (SEQ ID NO: 71) und DYMmu003 (SEQ ID NO: 72) umfasst.

## Claims

1. Method for genetically identifying and/or for distinguishing two or more animals of the species mouse, rat, hamster and zebrafish from different outbred strains, of the same outbred strain or of substrains of the same outbred strain, comprising the following substeps:
(a) using at least one DNA sample which is to be analysed;
(b) selecting a set of loci for the DNA sample which comprises at least 6, 7 or 8, particularly preferably at least 9 or 10, most preferably at least 11 autosomal STR markers which are coamplified simultaneously in a reaction mix, the autosomal STR loci being **characterized by** a high value for the "polymorphism information content", which is within the range of 0.50 to 0.99, particularly preferably within the range between 0.60 to 0.99;
(c) selecting a set of loci for the DNA sample which comprises at least two Y-chromosomal STR markers which are coamplified simultaneously with the autosomal STR loci (b) in a reaction mix;
(d) using a set of oligonucleotide primer pairs in which each primer pair binds specifically to the flanking DNA segments of an STR marker from the sets of STR loci (b) and (c);
(e) coamplifying the loci selected for the set, the reaction product being a mixture consisting of amplified alleles of each of the coamplified STR loci of the sets (b) and (c);
(f) analysing the allele mixture and determining and assigning the specific alleles for each of the amplified loci of the sets (b) and (c) for the given DNA sample.

2. Method according to Claim 1, **characterized in that** at least two of the at least 6 autosomal STR loci (b) are selected from the group comprising the loci D1Mmu121 (SEQ ID NO: 51), D2Mmu008 (SEQ ID NO: 52), D3Mmu158 (SEQ ID NO: 53), D4Mmu155 (SEQ ID NO: 54), D5Mmu108 (SEQ ID NO: 55), D6Mmu120 (SEQ ID NO: 56), D7Mmu003 (SEQ ID NO: 57), D8Mmu127 (SEQ ID NO: 58), D9Mmu100 (SEQ ID NO: 59), D10Mmu043 (SEQ ID NO: 60), D11Mmu030 (SEQ ID NO: 61), D12Mmu056 (SEQ ID NO: 62), D13Mmu096 (SEQ ID NO: 63), D14Mmu074 (SEQ ID NO: 64), D15Mmu084 (SEQ ID NO: 65), D16Mmu030 (SEQ ID NO: 66), D17Mmu041 (SEQ ID NO: 67), D18Mmu069 (SEQ ID NO: 68) and D19Mmu008 (SEQ ID NO: 69).

3. Method according to Claims 1 and 2, **characterized in that** the set of at least eleven autosomal loci in step (b) comprises the loci D2Mmu008 (SEQ ID NO: 52), D3Mmu158 (SEQ ID NO: 53), D4Mmu155 (SEQ ID NO: 54), D6Mmu120 (SEQ ID NO: 56), D7Mmu003 (SEQ ID NO: 57), D8Mmu127 (SEQ ID NO: 58), D10Mmu043 (SEQ ID NO: 60), D13Mmu096 (SEQ ID NO: 63), D14Mmu074 (SEQ ID NO: 64), D16Mmu030 (SEQ ID NO: 66) and D18Mmu069 (SEQ ID NO: 68).

4. Method according to Claim 1, further comprising sex identification in the DNA sample of the individual in step (a) on the basis of the set of loci according to step (c).

5. Method according to Claim 4, further comprising distinguishing between Y chromosomes of the type Mus musculus musculus and of the type Mus musculus domesticus on the basis of the set of loci according to step (c).

6. Method according to Claim 4, **characterized in that** the locus for sex determination is a polymorphic STR locus of mouse that can distinguish Y-chromosomal haplotypes and is selected from a group of loci listed as follows: DYMmu001 (SEQ ID NO: 70), DYMmu002 (SEQ ID NO: 71) and DYMmu003 (SEQ ID NO: 72).

7. Method according to Claim 4, **characterized in that** the locus for sex determination is a polymorphic STR locus of rat that can distinguish Y-chromosomal haplotypes and is selected from a group of loci listed as follows: DYRno004 (SEQ ID NO: 73 and SEQ ID NO: 74), DYRno165 (SEQ ID NO: 75) and DYRno304 (SEQ ID NO: 76).

8. Method according to Claim 1, **characterized in that** a multiplex amplification with at least thirteen pairs of oligonucleotide primers, which comprises at least eleven autosomal and two Y-chromosomal STR loci, is carried out.

9. Method according to Claim 1, **characterized in that** at least one oligonucleotide primer of a primer pair is covalently coupled to a detection dye, preferably to a fluorescent dye.

10. Method according to Claim 9, **characterized in that** at least four different dye-coupled primers with four different detection dyes, preferably four different fluorescent dyes, are used.

11. Method according to Claim 1, **characterized in that** the assignment of the amplified alleles in step (f) is done on the basis of comparison with a size standard, the size standard being a mixture of DNA fragments of known size and/or a locus-specific mixture of known alleles.

12. Kit for the genetic identification and/or for the distinguishing of two or more animals from DNA extracts of individuals from wild-type populations, from different inbred or outbred strains, of the same inbred or outbred strain or of substrains of the same inbred or outbred strain of mouse, comprising:
(a) a set of oligonucleotide primer pairs in which each primer pair binds specifically to the flanking DNA segments of a locus from the set of STR loci and which comprises at least 6, 7 or 8, particularly preferably at least 9 or 10, most preferably at least 11 autosomal STR markers which can be coamplified simultaneously in a reaction mix, the autosomal STR loci being **characterized by** a high value for the "polymorphism information content", which is within the range of 0.50 to 0.99, particularly preferably within the range between 0.60 to 0.99;
(b) a set of oligonucleotide primer pairs which bind specifically to the flanking DNA segments of the Y chromosome and which comprises at least two, preferably three Y-chromosomal STR markers which can be coamplified simultaneously with the autosomal STR loci (a) in a reaction mix;
(c) reagents which are sufficient for carrying out at least one multiplex PCR; and
(d) a size standard which contains different DNA markers of a known fragment length.

13. Kit according to Claim 12, **characterized in that** at least two of the at least 6 autosomal STR loci (a) are selected from the group comprising the loci D1Mmu121 (SEQ ID NO: 51), D2Mmu008 (SEQ ID NO: 52), D3Mmu158 (SEQ ID NO: 53), D4Mmu155 (SEQ ID NO: 54), D5Mmu108 (SEQ ID NO: 55), D6Mmu120 (SEQ ID NO: 56), D7Mmu003 (SEQ ID NO: 57), D8Mmu127 (SEQ ID NO: 58), D9Mmu100 (SEQ ID NO: 59), D10Mmu043 (SEQ ID NO: 60), D11Mmu030 (SEQ ID NO: 61), D12Mmu056 (SEQ ID NO: 62), D13Mmu096 (SEQ ID NO: 63), D14Mmu074 (SEQ ID NO: 64), D15Mmu084 (SEQ ID NO: 65), D16Mmu030 (SEQ ID NO: 66), D17Mmu041 (SEQ ID NO: 67), D18Mmu069 (SEQ ID NO: 68) and D19Mmu008 (SEQ ID NO: 69).

14. Kit according to Claim 12, **characterized in that** the set of at least eleven autosomal loci in step (a) comprises the loci D2Mmu008 (SEQ ID NO: 52), D3Mmu158 (SEQ ID NO: 53), D4Mmu155 (SEQ ID NO: 54), D6Mmu120 (SEQ ID NO: 56), D7Mmu003 (SEQ ID NO: 57), D8Mmu127 (SEQ ID NO: 58), D10Mmu043 (SEQ ID NO: 60), D13Mmu096 (SEQ ID NO: 63), D14Mmu074 (SEQ ID NO: 64), D16Mmu030 (SEQ ID NO: 66) and D18Mmu069 (SEQ ID NO: 68) .

15. Kit according to any of Claims 12 to 14, **characterized in that** the set of Y-chromosomal loci (b) comprises the loci DYMmu001 (SEQ ID NO: 70), DYMmu002 (SEQ ID NO: 71) and DYMmu003 (SEQ ID NO: 72).

## Revendications

1. Procédé pour l'identification génétique et/ou la distinction de deux animaux ou plus des espèces souris, rat, hamster et poisson zèbre à partir de différentes souches non consanguines, de la souche non consanguine identique ou de sous-souches de la souche non consanguine identique, comprenant les étapes partielles suivantes :
(a) utilisation d'au moins un échantillon d'ADN qui doit être analysé ;
(b) sélection d'un ensemble de loci pour l'échantillon d'ADN, qui comprend au moins 6, 7 ou 8, d'une manière particulièrement préférée au moins 9 ou 10, tout spécialement au moins 11 marqueurs STR autosomaux, qui sont simultanément co-amplifiés dans une masse réactionnelle, les loci STR autosomaux étant **caractérisés par** une valeur élevée du "polymorphism information content", comprise dans la plage de 0,50 à 0,99, d'une manière particulièrement préférée dans la plage entre 0,60 et 0,99 ;
(c) sélection d'un ensemble de loci pour l'échantillon d'ADN, qui comprend au moins deux marqueurs STR du chromosome Y, qui sont simultanément amplifiés avec les loci STR autosomaux (b) dans une masse réactionnelle ;
(d) utilisation d'un ensemble de paires d'amorces oligonucléotidiques, chaque paire d'amorces se liant spécifiquement aux segments d'ADN flanquants d'un marqueur STR de l'ensemble des loci STR (b) et (c) ;
(e) co-amplification des loci sélectionnés pour l'ensemble, le produit de réaction représentant un mélange constitué d'allèles amplifiés de chacun des loci STR co-amplifiés des ensembles (b) et (c) ;
(f) analyse du mélange d'allèles et détermination et affectation des allèles spécifiques pour chacun des loci amplifiés des ensembles (b) et (c) pour l'échantillon d'ADN donné.

2. Procédé selon la revendication 1, **caractérisé en ce qu'**au moins deux des au moins 6 loci STR autosomaux (b) sont choisis dans le groupe comprenant les loci D1Mmu121 (SEQ ID NO : 51), D2Mmu008 (SEQ ID NO : 52), D3Mmu158 (SEQ ID NO : 53), D4Mmu155 (SEQ ID NO : 54), D5Mmu108 (SEQ ID NO : 55), D6Mmu120 (SEQ ID NO : 56), D7Mmu003 (SEQ ID NO : 57), D8Mmu127 (SEQ ID NO : 58), D9Mmu100 (SEQ ID NO : 59), D10Mmu043 (SEQ ID NO : 60), D11Mmu030 (SEQ ID NO : 61), D12Mmu056 (SEQ ID NO : 62), D13Mmu096 (SEQ ID NO : 63), D14Mmu074 (SEQ ID NO : 64), D15Mmu084 (SEQ ID NO : 65), D16Mmu030 (SEQ ID NO : 66), D17Mmu041 (SEQ ID NO : 67), D18Mmu069 (SEQ ID NO : 68) et D19Mmu008 (SEQ ID NO : 69).

3. Procédé selon la revendication 1 et 2, **caractérisé en ce que** l'ensemble d'au moins onze loci autosomaux de l'étape (b) comprend les loci D2Mmu008 (SEQ ID NO : 52), D3Mmu158 (SEQ ID NO : 53), D4Mmu155 (SEQ ID NO : 54), D6Mmu120 (SEQ ID NO : 56), D7Mmu003 (SEQ ID NO : 57), D8Mmu127 (SEQ ID NO : 58), D10Mmu043 (SEQ ID NO : 60), D13Mmu096 (SEQ ID NO : 63), D14Mmu074 (SEQ ID NO : 64), D16Mmu030 (SEQ ID NO : 66) et D18Mmu069 (SEQ ID NO : 68) .

4. Procédé selon la revendication 1, comprenant en outre l'identification du sexe dans l'échantillon d'ADN de l'individu de l'étape (a) à l'aide de l'ensemble de loci selon l'étape (c).

5. Procédé selon la revendication 4, comprenant en outre la distinction des chromosomes Y du type Mus musculus musculus et du type Mus musculus domesticus à l'aide de l'ensemble de loci selon l'étape (c).

6. Procédé selon la revendication 4, **caractérisé en ce que** le locus destiné à la détermination du sexe représente un locus STR polymorphe de souris, qui peut distinguer des haplotypes du chromosome Y et est choisi dans un groupe des loci listés ci-après : DYMmu001 (SEQ ID NO : 70), DYMmu002 (SEQ ID NO : 71) et DYMmu003 (SEQ ID NO : 72).

7. Procédé selon la revendication 4, **caractérisé en ce que** le locus destiné à la détermination du sexe représente un locus STR polymorphe de rat, qui peut distinguer des haplotypes du chromosome Y et est choisi dans un groupe des loci listés ci-après : DYRno004 (SEQ ID NO : 73 et SEQ ID NO : 74), DYRno165 (SEQ ID NO : 75) et DYRno304 (SEQ ID NO : 76).

8. Procédé selon la revendication 1, **caractérisé en ce qu'**on procède à une amplification multiplexe avec au moins treize paires d'amorces oligonucléotidiques, qui comprennent au moins onze loci STR autosomaux et deux loci STR du chromosome Y.

9. Procédé selon la revendication 1, **caractérisé en ce qu'**au moins une amorce oligonucléotidique d'une paire d'amorces est couplée par liaison covalente à un colorant de détection, de préférence un colorant fluorescent.

10. Procédé selon la revendication 9, **caractérisé en ce qu'**au moins quatre amorces différentes couplées à un colorant sont utilisées avec quatre colorants de détection différents, de préférence quatre colorants fluorescents différents.

11. Procédé selon la revendication 1, **caractérisé en ce que** l'affectation des allèles amplifiés de l'étape (f) est réalisée à l'aide d'une comparaison avec un étalon de taille, l'étalon de taille représentant un mélange de fragments d'ADN de taille connues et/ou un mélange, spécifique de locus, d'allèles connus.

12. Trousse pour l'identification génétique et/ou la distinction de deux animaux ou plus à partir d'extraits d'ADN d'individus de populations de type sauvage, à partir de différentes souches consanguines ou non consanguines, de la souche consanguine ou non consanguine identique ou de sous-souches de la souche consanguine ou non consanguine identique de souris, comprenant :
(a) un ensemble de paires d'amorces oligonucléotidiques, chaque paire d'amorces se liant spécifiquement aux fragments d'ADN flanquants d'un locus de l'ensemble des loci STR, et qui comprend au moins 6, 7 ou 8, d'une manière particulièrement préférée au moins 9 ou 10, tout spécialement au moins 11 marqueurs STR autosomaux, qui peuvent être simultanément co-amplifiés dans une masse réactionnelle, les loci STR autosomaux étant **caractérisés par** une valeur élevée du "polymorphism information content", qui est comprise dans la plage de 0,50 à 0,99, d'une manière particulièrement préférée dans la plage entre 0,60 et 0,99 ;
(b) un ensemble de paires d'amorces oligonucléotidiques qui se lient spécifiquement aux segments d'ADN flanquants du chromosome Y et qui comprend au moins deux, de préférence trois marqueurs STR du chromosome Y, qui peuvent être simultanément co-amplifiés avec les loci STR autosomaux (a) dans une masse réactionnelle ;
(c) des réactifs qui sont suffisants pour mettre en oeuvre au moins une PCR multiplexe ; et
(d) un étalon de taille, qui contient plusieurs marqueurs d'ADN ayant une longueur de fragment connue.

13. Trousse selon la revendication 12, **caractérisée en ce qu'**au moins deux des 6 loci STR autosomaux (a) sont choisis dans le groupe comprenant les loci D1Mmu121 (SEQ ID NO : 51), D2Mmu008 (SEQ ID NO : 52), D3Mmu158 (SEQ ID NO : 53), D4Mmu155 (SEQ ID NO : 54), D5Mmu108 (SEQ ID NO : 55), D6Mmu120 (SEQ ID NO : 56), D7Mmu003 (SEQ ID NO : 57), D8Mmu127 (SEQ ID NO : 58), D9Mmu100 (SEQ ID NO : 59), D10Mmu043 (SEQ ID NO : 60), D11Mmu030 (SEQ ID NO : 61), D12Mmu056 (SEQ ID NO : 62), D13Mmu096 (SEQ ID NO : 63), D14Mmu074 (SEQ ID NO : 64), D15Mmu084 (SEQ ID NO : 65), D16Mmu030 (SEQ ID NO : 66), D17Mmu041 (SEQ ID NO : 67), D18Mmu069 (SEQ ID NO : 68) et D19Mmu008 (SEQ ID NO : 69).

14. Trousse selon la revendication 12, **caractérisée en ce que** l'ensemble d'au moins onze loci autosomaux de l'étape (a) comprend les loci D2Mmu008 (SEQ ID NO : 52), D3Mmu158 (SEQ ID NO : 53), D4Mmu155 (SEQ ID NO : 54), D6Mmu120 (SEQ ID NO : 56), D7Mmu003 (SEQ ID NO : 57), D8Mmu127 (SEQ ID NO : 58), D10Mmu043 (SEQ ID NO : 60), D13Mmu096 (SEQ ID NO : 63), D14Mmu074 (SEQ ID NO : 64), D16Mmu030 (SEQ ID NO : 66) et D18Mmu069 (SEQ ID NO : 68) .

15. Trousse selon l'une des revendications 12 à 14, **caractérisée en ce que** l'ensemble des loci du chromosome Y (b) comprend les loci DYMmu001 (SEQ ID NO : 70), DYMmu002 (SEQ ID NO : 71) et DYMmu003 (SEQ ID NO : 72).
